# EUROPEAN PATENT APPLICATION

(11) **EP 4 725 966 A1**
(43) Date of publication of application: **15.04.2026**
(21) Application number: 24822643.3
(22) Date of filing: 07.06.2024
(51) Int. Cl.: C07K 16/28, C12N 15/13, A61K 47/68, A61K 39/395, A61K 45/00, A61K 31/537, A61K 38/07, A61P 35/00

(54) **HUMAN EPIDERMAL GROWTH FACTOR 3 ANTIBODY, PREPARATION METHOD THEREFOR, AND USE THEREOF**

(30) Priority: 13.06.2023 CN 202310694043
(71) Applicant: SHANGHAI RUOTUO BIOSCIENCES CO., LTD., Shanghai 200438 (CN)
(72) Inventor: HE, Honglin, Shanghai 200438 (CN); XU, Yanghua, Shanghai 200438 (CN); YE, Li, Shanghai 200438 (CN); SHI, Wenli, Shanghai 200438 (CN); XIA, Aikun, Shanghai 200438 (CN); SONG, Ning, Shanghai 200438 (CN); ZHONG, Ziyang, Shanghai 200438 (CN)
(74) Representative: Canzler & Bergmeier Patentanwälte Partnerschaft mbB
(86) International application number: PCT/CN2024/097922
(87) International publication number: WO 2024/255692

(57) **Abstract**

Provided are an antibody against human epidermal growth factor 3 (HER3), a preparation method, and a use thereof. The anti-HER3 antibody specifically binds to HER3 and is capable of being internalized by cells. The anti-HER3 antibody can inhibit diseases associated with HER3 expression (including overexpression) or those mediated by HER3, such as tumors.

## Description

### REFERENCE TO RELATED APPLICATIONS

This application claims priority to Chinese Patent Application No. CN202310694043.3, filed June 13, 2023.

### FIELD OF TECHNOLOGY

The present disclosure relates to the technical field of biomedicine, and particularly to an antibody against human epidermal growth factor 3, preparation method, and use thereof.

### BACKGROUND

HER3 (also referred to as ERRB3) is a member of the human epidermal growth factor receptor (EGFR) family, which also includes EGFR (ERRB1, also known as HER1), HER2 (ERRB2), and HER4 (ERRB4). HER3 includes an extracellular domain that is organized into four distinct domains, designated as Domains I, II, III, and IV, respectively. Domains I and III are leucine-rich β-helical regions that are involved in ligand binding. Domains II and IV are cysteine-rich regions, and Domain II also contains a dimerization arm necessary for interaction with other receptors. The intracellular domain of HER3 includes a juxta membrane region, a kinase domain, and a C-terminal tail.

Ligands for HER3 include, for example, neuregulin 1 (NRG-1) and neuregulin 2 (NRG-2). Upon ligand binding, HER3 is generally incapable of forming homodimers and instead primarily forms heterodimers with another receptor, leading to intracellular phosphorylation events. Furthermore, HER3 exhibits little or no intrinsic intracellular tyrosine kinase activity. Therefore, HER3 is unable to independently initiate downstream signaling pathways. Accordingly, HER3 typically forms heterodimers with other members of the EGFR family (e.g., EGFR and HER2) or with non-EGFR family receptors (e.g., the MET receptor and FGFR2) to participate in signal transduction and initiate pathways associated with cell proliferation or differentiation. HER3 preferentially forms heterodimers with EGFR and HER2 and exhibits lower affinity for HER4. In particular, the heterodimer formed by HER3 and HER2 are capable of activating the phosphatidylinositol 3-kinase (PI3K) pathway. Activation of the PI3K/AKT signaling pathway plays an important role in cancer cell survival and has been implicated in the development of therapeutic resistance.

HER3 has been reported to at a high expression level in a variety types of cancer, including breast cancer, colorectal cancer, lung cancer, pancreatic cancer, skin cancer, gastric cancer, ovarian cancer, and melanoma. Elevated expression of HER3 has also been evidenced being associated with disease progression and poor prognosis. In contrast to other members of the EGFR family, overexpression of HER3 alone is generally not oncogenic. However, the heterodimer formed by HER3 and HER2 plays an important role in the development of multiple types of cancer. Studies have shown that upregulation of HER3 expression represents one mechanism by which tumor cells evade inhibition of EGFR family tyrosine kinase inhibitors. In addition, signal transduction mediated by the HER2-HER3 heterodimer has been shown being associated with resistance to EGFR tyrosine kinase inhibitors (TKIs), such as gefitinib, in head and neck cancer and breast cancer. In colorectal cancer, increased levels of neuregulin 1 (NRG1, the HER3 ligand), as well as enhanced formation of the HER2-HER3 heterodimer, have been reported to confer resistance to EGFR-targeting antibodies, such as cetuximab. Accordingly, HER3 is considered an attractive therapeutic target for cancer treatment.

The development of monoclonal antibodies targeting HER3 has been proposed as a potential therapeutic approach for the treatment of tumors with high EGFR or HER2 expression that relapse after prior therapy. Because HER3 lacks substantial intrinsic kinase activity, anti-HER3 antibodies are generally designed to exert their effects by blocking interactions between HER3 and its ligands, or between HER3 and other receptors in the EGFR family, and/or by recruiting immune effector cells to mediate cancer cell killing. To date, although several anti-HER3 antibodies are under clinical development or preclinical evaluation, no anti-HER3 antibody has yet been approved for marketing. In addition, HER3-directed bispecific antibodies and antibody-drug conjugates (ADCs) are also being evaluated in clinical trials. Among these, the antibody-drug conjugate patritumab deruxtecan, developed by Daiichi Sankyo, is currently undergoing Phase III clinical evaluation. Patritumab deruxtecan includes an anti-HER3 antibody, patritumab, conjugated to a small-molecule payload, DXd. Based on available clinical trial data, patritumab deruxtecan has demonstrated therapeutic activity in patients with non-small cell lung cancer who have developed resistance to EGFR inhibitors. Nevertheless, as the importance of HER3 in tumor progression and therapeutic resistance becomes increasingly evident, there remains a significant need for the development of effective therapeutics targeting HER3.

### SUMMARY

The present disclosure provides an antibody against human epidermal growth factor 3, a preparation method, and a use thereof.

In a first aspect, the present disclosure provides an anti-HER3 antibody, including a light chain variable region and a heavy chain variable region.

The amino acid sequence of CDR1 of the light chain variable region is one of SEQ ID NOs:21, 30, 12, 18, and 27.

The amino acid sequence of CDR2 of the light chain variable region is one of SEQ ID NOs:22, 31, 13, and 28.

The amino acid sequence of CDR3 of the light chain variable region is one of SEQ ID NOs:23, 32, 14, and 19.

The amino acid sequence of CDR1 of the heavy chain variable region is one of SEQ ID NOs:24, 33, and 15.

The amino acid sequence of CDR2 of the heavy chain variable region is one of SEQ ID NOs:25, 34, and 16.

The amino acid sequence of CDR3 of the heavy chain variable region is one of SEQ ID NOs:26, 35, 17, 20, and 29.

In some embodiments, the anti-HER3 antibody is an antibody (a), which may be a humanized (h)HE4 antibody, a mouse (m)HE4 antibody, or a chimeric (c)HE4 antibody as shown in embodiments. For antibody (a), the amino acid sequences of CDR1, CDR2, and CDR3 of the light chain variable region are as shown in SEQ ID NOs:21, 22, and 23, respectively; and the amino acid sequences of CDR1, CDR2, and CDR3 of the heavy chain variable region of the antibody (a) are as shown in SEQ ID NOs:24, 25, and 26, respectively.

In some embodiments, the anti-HER3 antibody is an antibody (c), which may be a humanized HE6 antibody, a mouse HE6 antibody, or a chimeric HE6 antibody as shown in embodiments. For antibody (c), the amino acid sequences of CDR1, CDR2, and CDR3 of the light chain variable region are as shown in SEQ ID NO:30, 31, and 32, respectively; and the amino acid sequences of CDR1, CDR2, and CDR3 of the heavy chain variable region are as shown in SEQ ID NOs:33, 34, and 35, respectively.

In some embodiments, the anti-HER3 antibody is an antibody (d), which may be a humanized HE1 antibody, a mouse HE1 antibody, or a chimeric HE1 antibody as shown in embodiments. For antibody (d), the amino acid sequences of CDR1, CDR2, and CDR3 of the light chain variable region are as shown in SEQ ID NOs:12, 13, and 14, respectively; and the amino acid sequences of CDR1, CDR2, and CDR3 of the heavy chain variable region are as shown in SEQ ID NOs:15, 16, and 17, respectively.

In some embodiments, the anti-HER3 antibody is an antibody (e), which may be a humanized HE2 antibody, a mouse HE2 antibody, or a chimeric HE2 antibody as shown in embodiments. For antibody (e), the amino acid sequences of CDR1, CDR2, and CDR3 of the light chain variable region are as shown in SEQ ID NOs:18, 13, and 19, respectively; and the amino acid sequences of CDR1, CDR2, and CDR3 of the heavy chain variable region are as shown in SEQ ID NOs:15, 16, and 20, respectively.

In some embodiments, the anti-HER3 antibody is an antibody (f), which may be a humanized HE5 antibody, a mouse HE5 antibody, or a chimeric HE5 antibody as shown in embodiments. For antibody (f), the amino acid sequences of CDR1, CDR2, and CDR3 of the light chain variable region are as shown in SEQ ID NOs:27, 28, and 19, respectively; and the amino acid sequences of CDR1, CDR2, and CDR3 of the heavy chain variable region are as shown in SEQ ID NOs:15, 16, and 29, respectively.

In some embodiments, the anti-HER3 antibody includes a monoclonal antibody, a single-chain variable fragment antibody (scFv), a domain antibody, an Fab fragment, an Fd fragment, an Fv fragment, or an F(ab')₂ fragment.

In some embodiments, the anti-HER3 antibody is a humanized antibody, a chimeric antibody, or a murine antibody.

In some embodiments, the chimeric antibody includes a light chain variable region and a heavy chain variable region derived from a murine antibody. The heavy chain constant region of the chimeric antibody is replaced with a human antibody IgG1 heavy chain constant region, and the light chain constant region of the chimeric antibody is replaced with a human kappa light chain constant region.

In some embodiments, the humanized antibody is an antibody in which the complementarity-determining regions (CDRs) of a murine antibody are retained, while the framework regions (FRs) of the heavy and light chain variable regions are substituted with the corresponding human immunoglobulin germline-derived FR sequences that have the highest homology to the corresponding murine FRs. Preferably, the humanized antibody includes a human IgG1 heavy chain constant region and a human kappa light chain constant region.

In some embodiments, the light chain FR region of the humanized antibody includes: FR1 including residues 1-23 of SEQ ID NO:36, FR2 including residues 35-49 of SEQ ID NO:36, FR3 including residues 57-88 of SEQ ID NO:36, and FR4 including residues 98-108 of SEQ ID NO:36.

In some embodiments, the heavy chain FR region of the humanized antibody includes: FR1 including residues 1-27 of SEQ ID NO:37, FR2 including residues 36-47 of SEQ ID NO:37, FR3 including residues 60-96 of SEQ ID NO:37, and FR4 including residues 112-122 of SEQ ID NO:37.

In some embodiments, the antibody is a humanized antibody, including a light chain variable region with the amino acid sequence as shown in SEQ ID NO:36 and a heavy chain variable region with the amino acid sequence as shown in SEQ ID NO:37.

In some embodiments, the antibody includes a light chain variable region with the amino acid sequence as shown in SEQ ID NO:5 and a heavy chain variable region with the amino acid sequence as shown in SEQ ID NO:6. This antibody corresponds to the mHE4 antibody in the embodiments.

In some embodiments, the antibody includes a light chain variable region with the amino acid sequence as shown in SEQ ID NO:9 and a heavy chain variable region with the amino acid sequence as shown in SEQ ID NO:10. This antibody corresponds to mHE6 antibody in the embodiments.

In some embodiments, the antibody includes a light chain variable region with the amino acid sequence as shown in SEQ ID NO:9 and a heavy chain variable region with the amino acid sequence as shown in SEQ ID NO:11. This antibody corresponds to mHE7 antibody in the embodiments.

In some embodiments, the antibody includes a light chain variable region with the amino acid sequence as shown in SEQ ID NO:1 and a heavy chain variable region with the amino acid sequence as shown in SEQ ID NO:2. This antibody corresponds to mHE1 antibody in the embodiments.

In some embodiments, the antibody includes a light chain variable region with the amino acid sequence as shown in SEQ ID NO:3 and a heavy chain variable region with the amino acid sequence as shown in SEQ ID NO:4. This antibody corresponds to mHE2 antibody in the embodiments.

In some embodiments, the antibody includes a light chain variable region with the amino acid sequence as shown in SEQ ID NO:7 and a heavy chain variable region with the amino acid sequence as shown in SEQ ID NO:8. This antibody corresponds to mHE2 antibody in the embodiments.

In some embodiments, the antibody further includes a heavy chain variable region amino acid sequence having at least 85% identity (e.g., 88%, 90%, 93%, 95%, 97%, or 99% or higher) with any one of the aforementioned heavy chain variable region amino acid sequences, and a light chain variable region amino acid sequence having at least 85% identity (e.g., 88%, 90%, 93%, 95%, 97%, or 99% or higher) with any of the aforementioned light chain variable region amino acid sequences, and provided that such an antibody possesses the same function as the antibody described in the examples of the present disclosure.

In another aspect, the present disclosure provides a polynucleotide encoding the anti-HER3 antibody described above.

In another aspect, the present disclosure provides a construct (e.g., an expression vector) including the polynucleotide.

In another aspect, the present disclosure provides an antibody expression system, including the construct or a genome into which the exogenous polynucleotide encoding the anti-HER3 antibody described above is integrated.

In some embodiments, the antibody expression system is a cell-based expression system.

In another aspect, the present disclosure provides a method for preparing any of the anti-HER3 antibodies described above, including: performing expression using the antibody expression system under conditions suitable for expression, to obtain the antibody.

In some embodiments, the method further includes purifying and isolating the antibody.

In another aspect, the present disclosure provides a use of any of the anti-HER3 antibodies described above in the following:
(i) preparing an anti-tumor drug that specifically targets tumor cells expressing HER3;
(ii) preparing an antibody-drug conjugate or an immunoconjugate;
(iii) preparing a bispecific or multispecific antibody;
(iv) preparing a reagent for diagnosing a tumor that expresses HER3; or
(v) preparing immune cells modified with a chimeric antigen receptor.

In another aspect, the present disclosure provides an immunoconjugate, including any of the anti-HER3 antibodies described above, and a functional molecule connected thereto (including covalent linkage, conjugation, attachment, and adsorption).

In some embodiments, the functional molecule includes, but is not limited to, a tumor inhibitor; a molecule targeting tumor-associated surface markers; a cytotoxin; a radioactive isotope; a biologically active protein; a molecule or a detectable label, each targeting immune cell surface markers; an extracellular hinge region, a transmembrane region and an intracellular signaling region based on chimeric antigen receptor technology; or a combination thereof.

In some embodiments, the tumor inhibitor is an anti-tumor toxin or an antitumor cytokine.

In some embodiments, the molecule targeting tumor-associated surface markers is an antibody or a ligand that binds to tumor-associated surface markers.

In some embodiments, the molecule targeting immune cell surface markers is an antibody or a ligand that binds to immune cell surface markers.

In some embodiments, the anti-tumor toxin includes, but is not limited to, a toxin acting on microtubules; a toxin acting on DNA or a derivative thereof; or a compound or a derivative thereof that acts on intracellular metabolism, transcription, translation, or signal transduction.

In some embodiments, the anti-tumor cytokine includes, but is not limited to, IL-2, IL-12, IL-15, IFN-beta, TNF-alpha, or a variant thereof.

In some embodiments, the antibody that binds to tumor-associated surface markers is an antibody recognizing an antigen other than HER3. The other antigens include EGFR, EGFRvIII, mesothelin, HER2, EphA2, cMet, EpCAM, MUC1, MUC16, CEA, Claudin 18.2, Claudin 6, WT1, NY-ESO-1, MAGE 3, CD47, ASGPR1, or CDH16.

In some embodiments, the antigen for the antibody that binds to immune cell surface markers includes CD3, CD4, CD8, PD-1, PD-L1, LAG-3, TIM-3, 4-1BB, OX40, ICOS, B7-H3, CD16, CTLA-4, TIGIT, VISTA, GITR, CD27, SIRPα, CD32, CD64, ILT2, ILT4, NKG2A, NKG2D, or KIR.

In some embodiments, the toxin acting on microtubules includes monomethyl auristatin or a derivative thereof, or maytansinoid or a derivative thereof.

In some embodiments, the toxin acting on DNA includes DXd or a derivative thereof, Duocarmycin, Calicheamicin, Pyrrolobenzodiazepines (PBDs), or SN-38 or a derivative thereof.

In some embodiments, the toxin further includes related compounds and their derivatives acting on other functions within tumor cells, such as metabolism, transcription, translation, and signal transduction.

In some embodiments, the anti-tumor toxin is connected to the antibody via a linker. The linker includes, but is not limited to, maleimide-GGFG peptide linker, maleimidocaproyl valine-citrulline para-amino benzyloxycarbonyl (mc-Val-Cit-PAB), or maleimidocaproyl valine-alanine para-amino benzyloxycarbonyl (mc-Val-Ala-PAB).

In some embodiments, the detectable label includes, but is not limited to, a fluorescent label or a chromogenic label.

In some embodiments, the intracellular signaling region includes, but is not limited to, a CD3ζ chain, an Fc ε RI γ immunoreceptor tyrosine-based activation motif (ITAM), or an intracellular signaling region of a costimulatory signaling molecule selected from CD27, CD28, CD137, CD134, MyD88, and CD40.

In some embodiments, the transmembrane region includes, but is not limited to, a transmembrane region of CD8 or CD28.

In another aspect, the present disclosure provides a pharmaceutical composition (including a diagnostic composition, such as a diagnostic reagent), including any of the anti-HER3 antibodies or any of the immunoconjugates described above.

In some embodiments, the pharmaceutical composition further includes a pharmaceutically acceptable carrier.

In another aspect, the present disclosure provides a use of the anti-HER3 antibody, the immunoconjugate, or the pharmaceutical composition described above in the preparation of a formulation, a kit, or a package for diagnosing or treating a HER3-expressing tumor.

In some embodiments, the tumor includes, but is not limited to, breast cancer, colon cancer, lung cancer, pancreatic cancer, skin cancer, gastric cancer, ovarian cancer, or melanoma.

In another aspect, the present disclosure provides a kit or a package, including the anti-HER3 antibody, the immunoconjugate, or the pharmaceutical composition described above.

Other aspects of the present disclosure will be apparent to those skilled in the art in view of the disclosure herein.

### BRIEF DESCRIPTION OF THE DRAWINGS

FIG. 1 shows the results of serum titer analysis of immunized mice as determined by ELISA and FACS according to the present disclosure.
FIG. 2 shows the ELISA results illustrating the binding affinity of the chimeric antibody of the present disclosure to human and cynomolgus monkey HER3.
FIG. 3 shows the FACS results illustrating the binding affinity of the chimeric antibody of the present disclosure to human and cynomolgus monkey HER3.
FIG. 4 shows the antigen-binding epitope results of the chimeric antibody of the present disclosure and patritumab.
FIG. 5 shows the internalization assay results of the chimeric antibody of the present disclosure.
FIG. 6 shows the cytotoxic activity of an antibody-drug conjugate based on the chimeric antibody of the present disclosure against tumor cells.
FIG. 7 shows the ELISA results indicating the binding affinity of the humanized antibody of the present disclosure to human and cynomolgus monkey HER3.
FIG. 8 shows the FACS results indicating the binding affinity of the humanized antibody of the present disclosure.
FIG. 9 shows the binding capabilities of the humanized antibody of the present disclosure and patritumab to different subdomains of HER3.
FIG. 10 shows the binding capabilities of the humanized antibodies of the present disclosure to EGFR, HER2, and HER4.
FIG. 11 shows the cellular imaging results of internalization of the humanized antibody of the present disclosure.
FIG. 12 shows the FACS analysis results of internalization of the humanized antibody of the present disclosure.
FIG. 13 shows the cytotoxic activity of an antibody-drug conjugate based on the humanized antibody of the present disclosure against tumor cells.
FIG. 14 shows the *in vivo* antitumor efficacy of an antibody-drug conjugate of the present disclosure in SW620 tumor-bearing mice.
FIG. 15 shows the *in vivo* antitumor efficacy of an antibody-drug conjugate of the present disclosure in DiFi tumor-bearing mice.
FIG. 16 shows the process from hybridoma generation to the production of the humanized antibody according to the present disclosure.

### DETAILED DESCRIPTION

The present disclosure provides an anti-HER3 antibody that specifically binds to HER3 and is capable of being internalized by cells. The anti-HER3 antibody can inhibit diseases associated with HER3 expression (including overexpression) or diseases mediated by HER3, such as tumors.

### Definitions

In the present disclosure, the terms "antibody" or "immunoglobulin" are used as general terms, including full-length antibodies, single-chain variable fragment antibodies, and all portions, domains, or fragments thereof (including but not limited to antigen-binding domains or fragments). Furthermore, the term "sequence" (e.g., in terms such as "immunoglobulin sequence," "antibody sequence," "single variable domain sequence," "VHH sequence," or "protein sequence") shall generally be understood to include both the relevant amino acid sequence and the nucleic acid or nucleotide sequence encoding said sequence, unless a more restricted interpretation is required herein.

The term "monoclonal antibody" refers to a preparation of antibody molecules with a single molecular composition. Monoclonal antibodies exhibit a single binding specificity and affinity for a specific epitope.

The term "humanized antibody" refers to a molecule having an antigen-binding site substantially derived from an immunoglobulin of a non-human species, and the remaining of the immunoglobulin structure of the molecule is based on the structure and/or sequence of a human immunoglobulin. The antigen-binding site may include a complete variable domain fused to a constant domain, or may only include complementarity-determining regions (CDRs), which may be grafted into appropriate framework regions within a variable domain. The antigen-binding site may be wild-type or may be modified by one or more amino acid substitutions, for example, to more closely resemble a human immunoglobulin. Some forms of humanized antibodies retain all CDR sequences. Other forms have one or more CDRs that are altered compared to the original antibody.

"Sequence identity" between two polypeptide sequences indicates the percentage of amino acids that are identical between the sequences. "Sequence similarity" indicates the percentage of amino acids that are identical or represent conservative amino acid substitutions. Methods for evaluating the degree of sequence identity between amino acid or nucleotide sequences are known to those skilled in the art. For example, amino acid sequence identity is typically measured using sequence analysis software. For example, the BLAST program from the NCBI database can be used to determine identity.

An "effective amount" of an agent refers to the amount necessary to cause a physiological change in the cells or tissues to which it is administered.

A "therapeutically effective amount" of an agent (e.g., a pharmaceutical composition) refers to an amount that, when administered at the required dose and for the required period of time, achieves a desired therapeutic or prophylactic outcome. A therapeutically effective amount of an agent may, for example, eliminate, reduce, delay, minimize, or prevent adverse effects of a disease.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). Preferably, the individual or subject is a human.

The term "pharmaceutical composition" refers to a formulation in which the biological activity of active ingredients contained therein is effective, without containing other components that are unacceptably toxic to the subject to whom the composition is administered.

A "pharmaceutically acceptable carrier" refers to any component in a pharmaceutical composition other than the active ingredient that is non-toxic to a subject. Pharmaceutically acceptable carriers include, but are not limited to, buffers, excipients, stabilizers, or preservatives.

The term "treatment/prevention" (and its grammatical variants) refers to an attempt to alter the natural course of a disease in the treated individual and clinical interventions undertaken for prophylaxis or during the course of a clinicopathological process. Desired effects of treatment include, but are not limited to, preventing the occurrence or recurrence of a disease, alleviating symptoms, reducing the direct or indirect pathological consequences of the disease, preventing metastasis, slowing disease progression, alleviating the disease state, achieving remission, and improving prognosis. In some embodiments, the antibodies disclosed herein are used to delay disease onset or slow the progression of a condition.

The term "detectable label" refers to a marker that can be attached to an antibody to determine the presence, absence, or amount of a specific target in a to-be-tested sample. The detectable label may include, but is not limited to, an enzyme, a fluorescent label, a radionuclide, a quantum dot, colloidal gold, etc. More specifically, it may be selected from: horseradish peroxidase (HRP), alkaline phosphatase (AP), glucose oxidase, β-D-galactosidase, urease, catalase, or glucoamylase.

In the present disclosure, the tumor is a HER3-expressing tumor, preferably including but not limited to: breast cancer, colon cancer, lung cancer, pancreatic cancer, skin cancer, gastric cancer, ovarian cancer, melanoma, or a combination thereof.

### Antibodies

The present disclosure provides an anti-HER3 antibody. The anti-HER3 antibody includes light chain CDR1-CDR3 and heavy chain CDR1-CDR3 as listed in Table 6 or Table 3. In a preferred embodiment, the anti-HER3 antibody includes a light chain variable region and a heavy chain variable region as shown in Table 6 or Table 2. The present disclosure also includes antibodies in which the amino acid sequence of the heavy chain variable region exhibits at least 85% identity (e.g., 88%, 90%, 93%, 95%, 97%, 99%, or higher) with a sequence shown in Table 6 or Table 2, and the amino acid sequence of the light chain variable region exhibits at least 85% identity (e.g., 88%, 90%, 93%, 95%, 97%, 99%, or higher) with a sequence shown in Table 6 or Table 2, while retaining the same function or similar activity as the antibody described in the examples of the present disclosure.

The antigen-binding properties of an antibody are typically determined by three complementarity-determining regions (CDRs). These CDR regions, along with the framework regions (FR), are organized in an ordered manner, with the FR regions not directly participating in the binding interaction. The CDRs form loop structures that are brought into close spatial proximity through the β-sheets formed by the intervening FR regions, collectively constituting the antigen-binding site of the antibody. The CDR regions are of particular immunological interest. The antibodies of the present disclosure include modifications to both the CDR regions and the framework regions.

The anti-HER3 antibody of the present disclosure can efficiently and specifically bind to human HER3 or cynomolgus monkey HER3. The anti-HER3 antibody can efficiently block the function of HER3 and facilitate the delivery of a conjugated substance to target cells. After binding to HER3 on the cell surface, the antibody can be rapidly internalized into the cell.

The present disclosure also provides an anti-HER3 antibody-based fusion protein, including a first domain and a second domain. The first domain is the antibody described herein, and the second domain includes a fragment that extends the *in vivo* half-life and/or enhances binding to effector cells. The fusion protein may be a binding molecule capable of specifically binding to cells that express HER3.

In the second domain, the fragment that extends *in vivo* half-life may include serum albumin or a fragment thereof, polyethylene glycol, a domain that binds serum albumin (such as an anti-serum albumin antibody), a polyethylene glycol-liposome complex, or the like. The fragment that enhances binding to effector cells may include an immunoglobulin Fc region, preferably a human immunoglobulin Fc region. The human immunoglobulin Fc region may contain mutations that alter Fc-mediated effector functions, and the effector functions include one or more of CDC activity, ADCC activity, and ADCP activity. The immunoglobulin may be one or more of IgG, IgA1, IgA2, IgD, IgE, and IgM. In particular, IgG may be one or more of IgG1, IgG2, IgG3, and IgG4. The Fc region contained in the fusion protein enables dimer formation and extends the *in vivo* half-life of the fusion protein, while enhancing Fc-mediated effector functions. In one embodiment, the Fc region is the Fc region of human IgG1, preferably a wild-type human IgG1 Fc sequence. The Fc region may further include mutations that alter Fc-mediated effector functions, including a) CDC activity, b) ADCC activity, and c) ADCP activity. These mutations are described, for example, in Leonard G Presta, Current Opinion in Immunology, 2008, 20:460-470; Esohe E. Idusogie et al., J Immunol, 2000, 164:4178-4184; Raphael A. Clynes et al., Nature Medicine, 2000, Volume 6, Number 4:443-446; Paul R. Hinton et al., J Immunol, 2006, 176:346-356.

In the anti-HER3 antibody-based fusion protein of the present disclosure, a linker peptide may be provided between the first domain and the second domain. The linker peptide may be a flexible polypeptide chain including alanine (A), and/or serine (S), and/or glycine (G). The linker peptide may have a length of 3 to 30 amino acids, preferably 3 to 9 amino acids, 9 to 12 amino acids, 12 to 16 amino acids, or 16 to 20 amino acids. In one embodiment, the linker peptide may have a length of 8 or 15 amino acids.

The present disclosure also provides an isolated polynucleotide encoding the anti-HER3 antibody of the present disclosure or encoding the fusion protein of the present disclosure. The polynucleotide may be RNA, DNA, cDNA, or the like. Methods for providing such isolated polynucleotides are well known to those skilled in the art. For example, the polynucleotide may be prepared by automated DNA synthesis and/or recombinant DNA techniques, or may be isolated from an appropriate natural source.

### Constructs and Antibody Expression Systems

The present disclosure further provides a construct including the isolated polynucleotide described herein. Methods for constructing such a construct are well known to those skilled in the art. For example, the construct may be prepared *by in vitro* recombinant DNA techniques, DNA synthesis techniques, or *in vivo* recombination techniques. More specifically, the construct may be generated by inserting the isolated polynucleotide into a multiple cloning site of an expression vector. The expression vectors used in the present disclosure generally refer to various commercially available expression vectors well known in the art, including but not limited to bacterial plasmids, bacteriophages, yeast plasmids, plant cell viruses, mammalian cell viruses (such as adenoviruses or retroviruses), or other suitable vectors. The vector may further include one or more regulatory sequences operably linked to the polynucleotide sequence. The regulatory sequences may include a suitable promoter sequence. The promoter sequence is typically operably linked to the coding sequence of the amino acid sequence to be expressed. The promoter may be any nucleotide sequence that exhibits transcriptional activity in the selected host cell, including mutated, truncated, or hybrid promoters, and may be obtained from genes encoding extracellular or intracellular polypeptides that are homologous or heterologous to the host cell. The regulatory sequences may further include an appropriate transcription terminator sequence that is recognized by the host cell to terminate transcription. The terminator sequence is operably linked to the 3' end of the nucleotide sequence encoding the polypeptide, and any terminator that is functional in the selected host cell may be used in the present disclosure.

Suitable vectors generally include an origin of replication, a promoter sequence, convenient restriction enzyme sites, and one or more selectable markers that are functional in at least one organism. For example, such promoters may include, but are not limited to, the *Escherichia coli* lac or trp promoter; the bacteriophage λ PL promoter; and eukaryotic promoters, including the cytomegalovirus (CMV) immediate-early promoter, the herpes simplex virus (HSV) thymidine kinase promoter, simian virus 40 (SV40) early and late promoters, the methanol oxidase promoter of *Pichia pastoris,* and other promoters known in the art that are capable of controlling gene expression in prokaryotic or eukaryotic cells or viruses thereof. Marker genes may be used to confer a phenotypic trait for the selection of transformed host cells. Such marker genes may include, but are not limited to, dihydrofolate reductase, neomycin resistance, or green fluorescent protein (GFP) for eukaryotic cell culture, or tetracycline resistance or ampicillin resistance for *Escherichia coli.* When the polynucleotide is expressed, the expression vector may further include an enhancer sequence. Insertion of an enhancer sequence into the vector can enhance transcription. An enhancer is a cis-acting DNA element, typically about 10 to 300 base pairs in length, that acts on a promoter to increase gene transcription.

The present disclosure further provides an antibody expression system including the construct of the present disclosure, or a genome into which an exogenous polynucleotide of the present disclosure is integrated. Any cell suitable for expression from an expression vector may be used as a host cell. For example, the host cell may be a prokaryotic cell, such as a bacterial cell, or a eukaryotic cell, such as a yeast cell, or a mammalian cell. Specific examples for the host cell include, but are not limited to, bacterial cells, such as *Escherichia coli, Streptomyces, Salmonella typhimurium;* fungal cells such as yeast, filamentous fungi; plant cells; insect cells such as *Drosophila* S2 cells or Sf9 cells; animal cells such as CHO cells, COS cells, HEK293 cells, or Bowes melanoma cells, or any combination thereof. Methods for constructing such expression systems are well known to those skilled in the art and may include, but are not limited to, microinjection, gene gun, electroporation, virus-mediated transformation, electron bombardment, calcium phosphate precipitation, or any combination thereof.

### Immunoconjugates

The present disclosure further provides an immunoconjugate including the anti-HER3 antibody or the fusion protein of the present disclosure. The immunoconjugate typically further includes a functional molecule linked to (including but not limited to covalent linkage, conjugation, attachment, or adsorption) the antibody or fusion protein. The functional molecule may include, but is not limited to, a detectable label, a cytotoxin, a radioactive isotope, a biologically active protein, a molecule targeting tumor-associated surface markers, a tumor-inhibiting molecule, a molecule targeting immune cell surface markers, or a combination thereof.

Methods for preparing the immunoconjugate are well known to those skilled in the art. For example, the antibody and/or fusion protein may be directly linked to a functional molecule, or indirectly linked via a spacer of suitable length. The linkage may be achieved by chemical crosslinking or by genetic engineering (fusion expression), thereby obtaining the immunoconjugate.

For therapeutic purposes, it may be desirable to conjugate the antibody and/or fusion protein to a therapeutic effector moiety, such as a radioactive moiety. Such radioactive moieties may include radioactive isotopes or radionuclides (e.g., ³H, ¹⁴C, ¹⁵N, ³³P, ³⁵S, ⁹⁰Y, ⁹⁹Tc, ¹¹¹In, ¹²³I, ¹²⁵I, ¹³¹I, ²⁰¹Tl, ²¹³Bi), toxins, or cytotoxic moieties such as cell growth inhibitors.

The immunoconjugate may include the anti-HER3 antibody or fusion protein of the present disclosure and a detectable label. The detectable label includes, but is not limited to, a fluorescent label, a chromogenic label, and a protein tag, such as an enzyme, a cofactor, a fluorescent material, a luminescent material, a bioluminescent material, a radioactive material, a positron-emitting metal, and a non-radioactive paramagnetic metal ion. More than one label may be included. For detection and/or analysis and/or diagnostic purposes, the particular label used to tag an antibody depends on the specific detection, analysis, and/or diagnostic technique and/or method employed, such as immunohistochemical staining of tissue samples, flow cytometry, and the like. Suitable labels for detection, analysis, and/or diagnostic techniques and/or methods known in the art are well understood by those skilled in the art.

The anti-HER3 antibody or fusion protein of the present disclosure may be conjugated with a labeling moiety (a labeled polypeptide) and subsequently used, for example, for diagnostic purposes. Suitable labeling moieties may be selected from radioactive isotopes described above or moieties including radioactive isotopes or radionuclides; fluorescent moieties (such as fluorescent proteins including GFP, RFP, and the like; dyes; rhodamine; fluorescein and derivatives thereof such as FITC; cyanine dyes); enzyme moieties (such as horseradish peroxidase, alkaline phosphatase, or β-galactosidase); chemiluminescent moieties; biotin moieties; metal particles (such as gold particles); magnetic particles (such as particles having a core including magnetite (Fe₃O₄) and/or maghemite (Fe₂O₃)); predetermined peptide moieties; and the like.

The immunoconjugate may include the anti-HER3 antibody or fusion protein of the present disclosure and a molecule targeting immune cell surface markers. The molecule targeting immune cell surface markers may recognize immune cells and deliver the antibody of the present disclosure to the immune cells, subsequently, the antibody of the present disclosure directs the immune cells to tumor cells, thereby inducing immune cell-mediated tumor cell killing in addition to exerting the cytotoxic effects of the antibody itself.

The immunoconjugate may include the anti-HER3 antibody or fusion protein of the present disclosure and at least one molecule targeting tumor-associated surface markers or tumor-inhibiting molecule. The tumor-inhibiting molecule may be an anti-tumor cytokine or an anti-tumor toxin. For example, the cytokine may include, but is not limited to, IL-2, IL-12, IL-15, IFN-β, TNF-α, or the like. The molecule targeting tumor-associated surface markers may act synergistically with the antibody of the present disclosure to more precisely target tumor cells.

The immunoconjugate may further be a chimeric antigen receptor (CAR), which may be expressed on immune cells. The terms "immune cells" and "immune effector cells" are used interchangeably herein and include, but are not limited to, T lymphocytes, NK cells, and NKT cells, preferably NK cells and T lymphocytes. The chimeric antigen receptor generally includes, in sequence, an extracellular binding domain, a transmembrane domain, and an intracellular signaling domain. The extracellular binding domain includes the antibody or fusion protein of the present disclosure. The design of the transmembrane domain and intracellular signaling domain based on CAR technology is well known in the art. For example, the transmembrane domain may be a transmembrane domain of CD8 molecule or CD28 molecule, and the intracellular signaling domain may include an immunoreceptor tyrosine-based activation motif (ITAM) of the CD3ζ chain or the FcεRIγ chain, or an intracellular signaling domain of a costimulatory molecule (such as CD28, CD27, CD137, CD134, MyD88, CD40, and the like). More specifically, for T lymphocytes, first-generation CAR-T lymphocytes contain an intracellular signaling domain of only an ITAM, with the CAR components linked in the form scFv-TM-ITAM, thereby eliciting anti-tumor cytotoxic effects. Second-generation CAR T lymphocytes further include an intracellular signaling domain of CD28 or CD137 (also known as 4-1BB). The components of the chimeric antigen receptor are linked in the form of scFv-TM-CD28-ITAM or scFv-TM-CD137-ITAM. Costimulatory signaling via B7/CD28 or 4-1BBL/CD137 promotes sustained proliferation of T lymphocytes and enhances secretion of cytokines such as IL-2 and IFN-γ, while also improving *in vivo* persistence and anti-tumor efficacy of the CAR T cells. Third-generation CAR T lymphocytes include two costimulatory intracellular signaling domains. The components of the chimeric antigen receptor are linked in the form of scFv-TM-CD28-CD137-ITAM or scFv-TM-CD28-CD134-ITAM, which further enhances *in vivo* persistence and anti-tumor activity of the CAR T cells.

Accordingly, chimeric antigen receptors prepared using the anti-HER3 antibody or fusion protein of the present disclosure may include, in sequence, an extracellular binding domain, a transmembrane domain, and an intracellular signaling domain. The chimeric antigen receptor is selected from: the anti-HER3 antibody or fusion protein of the present disclosure, CD8, and CD3ζ; the anti-HER3 antibody or fusion protein of the present disclosure, CD8, CD137, and CD3ζ; the anti-HER3 antibody or fusion protein of the present disclosure, a transmembrane domain of CD28 (CD28a), an intracellular signaling domain of CD28 (CD28b), and CD3ζ; and the anti-HER3 antibody or fusion protein of the present disclosure, a transmembrane domain of CD28, an intracellular signaling domain of CD28, CD137, and CD3ζ.

By expressing the chimeric antigen receptor on the surface of immune effector cells, the immune effector cells may exhibit highly specific cytotoxic activity against HER3-expressing tumor cells, while simultaneously utilizing the tumor-killing effect mediated by the anti-HER3 antibody of the present disclosure.

As a preferred embodiment of the present disclosure, the anti-HER3 antibody of the present disclosure may be linked to a tumor-inhibiting molecule, preferably an anti-tumor toxin. Such anti-tumor toxins include, but are not limited to, DNA-acting toxins such as DXd, duocarmycin, calicheamicin, pyrrolobenzodiazepines (PBDs), SN-38, and related compounds and derivatives thereof; microtubule-acting toxins such as monomethyl auristatin and derivatives thereof, and maytansinoid and derivatives thereof; as well as compounds and derivatives thereof that act on other intracellular functions, including metabolism, transcription, translation, and signal transduction. The present disclosure further includes analogs, isomers, precursors, and the like of these small-molecule compounds used as toxins.

### Pharmaceutical Compositions and Kits

The present disclosure further provides a pharmaceutical composition including the anti-HER3 antibody of the present disclosure, or the fusion protein of the present disclosure, or the immunoconjugate of the present disclosure.

The pharmaceutical composition may further include one or more pharmaceutically acceptable carriers. Pharmaceutically acceptable carriers are non-toxic to the recipient at the employed dosages and concentrations, and may include, but are not limited to: buffers such as acetate, Tris, phosphate, citrate, and other organic acids; antioxidants including ascorbic acid and methionine; preservatives (such as stearyl dimethyl benzyl ammonium chloride; chlorhexidine; benzalkonium chloride; benzethonium chloride; phenol; butyl alcohol or benzyl alcohol; parabens such as methyl paraben or propyl paraben; catechol; resorcinol; cyclohexanol; 3-pentanol; and m-cresol); proteins such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrin; chelating agents such as EDTA; tonicity agents such as trehalose and sodium chloride; sugars such as sucrose, mannitol, trehalose, or sorbitol; surfactants such as polysorbates; counterions such as sodium; metal complexes (e.g., Zn-protein complexes); and/or non-ionic surfactants such as TWEEN^{®}, PLURONICS^{®}, or polyethylene glycol (PEG). Pharmaceutical formulations for *in vivo* administration are generally sterile, and methods for achieving sterility are well known to those skilled in the art, for example, sterile filtration through a sterilizing-grade membrane filter. Those skilled in the art may select appropriate pharmaceutically acceptable carriers according to the desired dosage form of the pharmaceutical composition, such that the pharmaceutical composition may be formulated into various forms, including but not limited to tablets, injections, or lyophilized formulations.

In the pharmaceutical composition, the fusion protein or immunoconjugate is generally present in an effective amount. The amount of the active ingredient corresponding to the effective amount may be determined according to the subject being treated and the specific route of administration. For example, based on the total mass of the pharmaceutical composition, the content of the fusion protein or immunoconjugate may range from about 0.01-99%, 0.1-70%, 1-30%, 0.01-0.05%, 0.05-0.1%, 0.1-0.3%, 0.3-0.5%, 0.5-1%, 1-3%, 3-5%, 5-10%, 10-20%, 20-30%, 30-50%, 50-70%, or 70-99%.

The fusion proteins, immunoconjugates, and pharmaceutical compositions of the present disclosure may be administered as a single active agent or in a combined therapy, that is, in combination with one or more other therapeutic agents. For example, the combined therapy may include administration of the fusion protein, immunoconjugate, or pharmaceutical composition of the present disclosure together with at least one additional anti-tumor agent. In another example, the combined therapy may include administration of the fusion protein, immunoconjugate, or pharmaceutical composition of the present disclosure together with an antibody targeting another tumor-specific antigen, such as, but not limited to, an anti-EGFR antibody, an anti-VEGF antibody, an anti-HER2 antibody, or an anti-Claudin18.2 antibody, or a combination thereof. Preferably, the inhibitor is a monoclonal antibody.

The present disclosure further provides a detection kit including the anti-HER3 antibody, fusion protein, or immunoconjugate of the present disclosure. The kit may further include, as needed, containers, controls (negative or positive controls), buffers, auxiliary reagents, and the like, which may be selected by those skilled in the art according to specific circumstances. The kit may further include instructions for use to facilitate operation by those skilled in the art.

The present disclosure further provides a method for detecting HER3 protein using the anti-HER3 antibody of the present disclosure, including but not limited to qualitative detection, quantitative detection, and localization detection. Specifically, the detection method includes, but is not limited to, immunofluorescence assay, immunohistochemistry assay, and radioimmunoassay.

**A** method for detecting the presence of HER3 protein in a sample may include contacting the sample with the anti-HER3 antibody of the present disclosure and observing whether an antibody complex is formed. The formation of the antibody complex indicates the presence of HER3 protein in the sample. The sample may be a cell and/or tissue sample; the sample may be fixed or dissolved in a medium; and the level of HER3 protein in the fixed or dissolved sample may be detected. In some embodiments, the to-be-detected object may be a cell-containing sample present in a cell preservation solution. In other embodiments, the antibody may further be conjugated with a fluorescent dye, chemical substance, polypeptide, enzyme, isotope, tag, or the like that is suitable for detection or detectable by other reagents.

### Uses

The present disclosure further provides a use of the anti-HER3 antibody, fusion protein, immunoconjugate, or pharmaceutical composition of the present disclosure in the preparation of a medicament for diagnosing, treating, or preventing diseases associated with cells expressing (or overexpressing) HER3.

**A** "therapeutically effective amount" of the fusion protein, immunoconjugate, or pharmaceutical composition of the present disclosure preferably results in a reduction in the severity of disease symptoms, an increase in the frequency and duration of asymptomatic periods, or prevention of injury or disability caused by disease-related suffering. For example, for the treatment of HER3-related tumors (including, but not limited to, ovarian cancer, endometrial cancer, breast cancer, cervical cancer, and the like), a "therapeutically effective amount" preferably inhibits cell growth or tumor growth by at least about 10%, at least about 20%, at least about 30%, at least about 40%, at least about 50%, at least about 60%, at least about 70%, or at least about 80%, as compared with an untreated subject. The ability to inhibit tumor growth may be evaluated in animal model systems that are predictive of efficacy against human tumors. Alternatively, the ability to inhibit tumor growth may be assessed by examining the ability to inhibit cell growth, which can be determined *in vitro* using assays well known to those skilled in the art. A therapeutically effective amount of the fusion protein, immunoconjugate, or pharmaceutical composition generally reduces tumor size or otherwise alleviates symptoms in the subject. Those skilled in the art may select an appropriate therapeutically effective amount based on practical considerations, such as the age of the subject, the severity of the subject's symptoms, and the particular composition or route of administration selected. Treatment regimens (e.g., dosage determination) may be determined by a physician, typically taking into account factors including, but not limited to, the disease being treated, individual patient condition, site of delivery, method of administration, and other relevant factors. A "prophylactically effective amount" refers to an amount that, when administered at the required dose and for the required period of time, effectively achieves the desired prophylactic effect. Generally, but not necessarily, because prophylactic dose is administered prior to disease onset or at an early stage of disease, a "prophylactically effective amount" is typically lower than a "therapeutically effective amount."

The beneficial technical effects of the present disclosure are as follows.

At present, no anti-HER3 monoclonal antibody has been approved for marketing, and based on current clinical trial results thus far, the therapeutic efficacy of the existing anti-HER3 monoclonal antibodies has not been compelling. The anti-HER3 antibodies of the present disclosure exhibit high affinity and desirable antigen-binding activity.

The anti-HER3 antibodies of the present disclosure can be rapidly and efficiently internalized by cells and can exert therapeutic effects in tumor treatment when formulated as antibody-drug conjugates (ADCs) or bispecific antibodies.

The antibodies of the present disclosure, such as hHE4-G1W, bind to a different epitope on HER3 compared with the reference antibody patritumab, thereby providing novel targeted antibodies for clinical applications.

Compared with the reference antibody patritumab, hHE4-G1W exhibits higher affinity for HER3-expressing tumor cells and a faster internalization rate, and when conjugated to small-molecule payloads, can bind with high affinity to HER3-expressing tumor cells, resulting in superior cell-killing effects.

The present disclosure is further illustrated by the following examples. It should be understood that these examples are provided for illustrative purposes only and are not intended to limit the scope of the present disclosure. Experimental methods not specifically described in the following examples are generally performed under conventional conditions, such as those described in Molecular Cloning: A Laboratory Manual, Third Edition, by J. Sambrook et al., Science Press, or in accordance with the instructions recommended by the respective manufacturers.

### Example 1: Preparation of Animals for Hybridoma Fusion

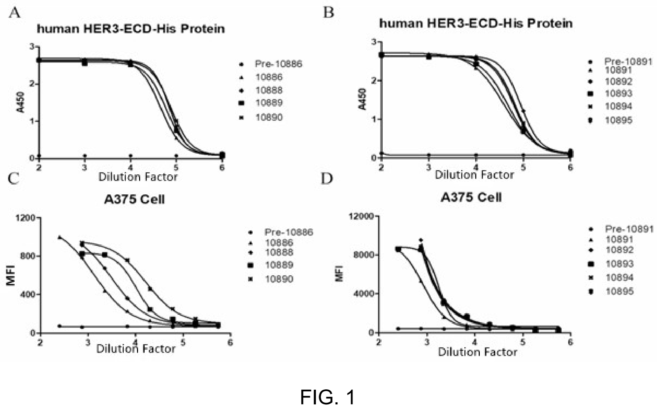

A fusion protein including the extracellular domain of human HER3 and a His tag (hHER3-ECD-His) was used as an immunogen. Multiple SJL mice, with identification numbers 10886, 10888, 10889, 10890, 10891, 10892, 10893, 10894, and 10895, were immunized via intraperitoneal injection to induce the production of antibodies against human HER3. Serum titers were evaluated by enzymelinked immunosorbent assay (ELISA). Firstly, hHER3-ECD-His protein was coated onto 96-well microplates. Serially diluted mouse sera were added and incubated at room temperature for 1 hour, followed by washing the microplates. An HRP-conjugated rabbit anti-mouse full-length secondary antibody (Thermo Fisher) was then added and incubated at room temperature for 1 hour. After washing, color development was carried out using TMB substrate, and absorbance was measured at 450 nm using a microplate reader. As shown in Panels A and B of FIG. 1, serum titers at the protein level reached levels higher than 1:1,000,000.

Serum titers were further evaluated by fluorescence activated cell sorting (FACS) using HER3-expressing A375 cells. The A375 cells were trypsinized to detach, collected by centrifugation, resuspended in PBS, counted, and transferred into 96-well plates. After centrifugation and removal of the supernatant, sera from the immunized mice were added and incubated at room temperature for 30 minutes. The cells were washed twice and then incubated with a PE-conjugated goat anti-mouse full-length secondary antibody (Jackson) in the dark for 30 minutes. After washing once, the cells were resuspended in PBS and analyzed by flow cytometry. As shown in Panels C and D of FIG. 1, mouse 10890 exhibited the highest serum titer at the cellular level, reaching approximately 182,000, while sera from the other mice showed titers of at least 1:20,000. These results demonstrated that antibodies present in the mouse sera specifically recognized HER3 expressed on the surface of A375 cells.

Based on the serum titer results, immunization of mice with the human HER3 extracellular domain protein was confirmed to have generated antibodies against human HER3. Mice 10889 and 10890 were selected for subsequent hybridoma fusion based on their titer results.

### Example 2: Isolation of Antibodies with High Affinity

Hybridoma cells capable of secreting anti-human-HER3 antibodies and exhibiting unlimited proliferation were generated by electrofusion of splenic lymphocytes isolated from the immunized mice and SP2/0 myeloma cells. Subcloning and screening of the resulting hybridoma cells were performed to obtain multiple antibodies specifically recognizing human HER3. For the obtained subcloned antibodies, ELISA and FACS analyses were carried out using the same methods as described in Example 1. As a result, five subcloned antibodies with relatively high affinity were selected. Human HER3 and cynomolgus monkey HER3 (Cyno HER3) were used as antigens, and the experimental results are shown in Table 1.

**Table 1**

| Antibody ID | Clone Number | ELISA Results (OD450) | | FACS Results (MFI) |
|---|---|---|---|---|
| | | human HER3 | Cyno HER3 | Human HER3-CHO |
| mHE1 | 161C2D1 | 1.86 | 1.77 | 173 |
| mHE2 | 167C5B3 | 1.96 | 1.77 | 195 |
| mHE4 | 180C1B1 | 2.07 | 1.87 | 199 |
| mHE5 | 184H5C5 | 1.90 | 1.82 | 224 |
| mHE6 | 197F6C4 | 2.12 | 1.51 | 205 |
| Blank Control | | 0.12 | 0.14 | 14 |

As shown in Table 1, the five subcloned antibodies specifically bound to both human HER3 and Cyno HER3 antigens and exhibited favorable binding performance.

### Example 3: Antibody Sequences

Messenger RNA (Omega) of the five hybridoma monoclonal cells obtained in Example 2 was extracted and was reverse-transcribed into cDNA (TAKARA). The genes encoding the variable regions of the antibody heavy chains and light chains were amplified from the cDNA by polymerase chain reaction (PCR), cloned into the pMD^{™}18-T vector (TAKARA), and chemically transformed into competent *E. coli* DH5α cells. Sequencing analysis was then performed to obtain the heavy chain and light chain sequences of the monoclonal antibodies. Table 2 and Table 3 list the amino acid sequences of the variable regions and the complementarity-determining regions (CDRs), respectively, of the mouse monoclonal antibodies against human HER3. Sequence analysis revealed that amino acid residues 73-75 ("NKS") in the heavy chain variable region of mHE6 constituted a potential glycosylation site. Accordingly, during subsequent construction of chimeric antibodies, the asparagine (N) residue at position 73 was mutated to aspartic acid (D). The resulting heavy chain was designated as mHE7 heavy chain. The light chain variable region of mHE7 was identical to that of mHE6.

**Table 2**

| Antibody Light Chain and Heavy Chain | Antibody Light Chain and Heavy Chain Variable Region Sequences |
|---|---|
| mHE1 Light Chain (SEQ ID NO:1) | DIVLTQSPASLAVSLGQRATISCRASESVDNYGIRLMHWYQQKSGQPPK LLIHRASNLESGIPARFSGSGSRTDFTLTINPVETDDVATYYCQQSNKDP FTFGSGTKLEIKR |
| mHE1 Heavy Chain (SEQ ID NO:2) | EVKLQESGPGLVKPSQSLSLTCSVTGYSITSGYYWNWIRQFPGNKLEW MGYISYDGSNNYNPSLKNRISITRDTSKNQFFLNLNSVTTEDTATYYCAR VDWDGDAMDYWGQGTSVTVSS |
| mHE2 Light Chain (SEQ ID NO:3) | DIVLTQSPASLAVSLGQRATISCRASESVDNYGISLMHWYQQKPGQPPK LLIYRASNLESGIPARFSGSGSRTDFTLTINPVETDDVATYYCQQSNKDPF TFGAGTKLELKR |
| mHE2 Heavy Chain (SEQ ID NO:4) | EVKLQESGPGLVKPSQSLSLTCSVTGYSITSGYYWNWIRQFPGNKLEW MGYISYDGSNNYNPSLKNRISITRDTSKNQFFLKLNSVTTEDTATYYCAR MDWDGDAMDYWGQGTSVTVSS |
| mHE4 Light Chain (SEQ ID NO:5) | DIQMTQSPASLSASVEETVTITCRASENIYSYLAWYQQKQGKSPQLLVYN AKTLAEGVPSRFSGSGSGTEFSLKINSLQPEDFGSYYCQNHYGTPYTF GGGTKLEIKQ |
| mHE4 Heavy Chain (SEQ ID NO:6) | EVQLVESGGGLVKPGGSLKLSCAASGFTFSSYVMSWVRQTPEKRLEWV ATISDGDSSTYYPDNVKGRFTISRDNAKNNLFLQMSHLKSEDTAMYFCA RDKGHGYDEGAFVYWGQGTLVTVSA |
| mHE5 Light Chain (SEQ ID NO:7) | DIVLTQSPASLAVSLGQRATISCRASESVDHYGSSFMHWYQQKPGQPPK LLIYRASNPESGIPARFSGSGSRTDFTLTINPVETDDVATYYCQQSNKDP PTFGGGTKLEIKR |
| mHE5 Heavy Chain (SEQ ID NO:8) | EVQLQESGPGLVKPSQSLSLTCSVTGYSITSGYYWNWIRQFPGNKLEW MGYISYDGSNNYNPSLKNRISITRDTSKNQFFLKLNSVTTEDTATYYCAS YDYDGDWFAYWGQGTLVTVSA |
| mHE6 Light Chain (SEQ ID NO:9) | DIELTQSPALMSASPGEKVTMTCSASSSVSYMYWYQQKPRSSPKPWIYL TSNLASGVPARFSGSGSGTSYSLTISSMEAEDAATYYCQQWSRNPLTFG AGTKLELKR |
| mHE6 Heavy Chain (SEQ ID NO:10) | EVQLQQSGPDLVKPGTSVKMSCKASGYTFTDYYMHWVRQSHGKSLEW IGYINPNNGDAIYNQKFKGKATLTVNKSSTTAYMALRSLTSEDSAVYFCAT YDYGGDFYFDVWGTGTTVTVSS |
| mHE7 Light Chain | (SEQ ID NO:9) |
| mHE7 Heavy Chain (SEQ ID NO:11) | EVQLQQSGPDLVKPGTSVKMSCKASGYTFTDYYMHWVRQSHGKSLEW IGYINPNNGDAIYNQKFKGKATLTVDKSSTTAYMALRSLTSEDSAVYFCAT YDYGGDFYFDVWGTGTTVTVSS |

**Table 3**

| Hybridoma Cell Line ID | | CDR1 | CDR2 | CDR3 |
|---|---|---|---|---|
| mHE1 | Light Chain | RASESVDNYGIRLMH (SEQ ID NO:12) | RASNLES (SEQ ID NO:13) | QQSNKDPFT (SEQ ID NO:14) |
| | Heavy Chain | SITSGYYWN (SEQ ID NO:15) | MGYISYDGSNN (SEQ ID NO:16) | ARVDWDGDAMDY (SEQ ID NO:17) |
| mHE2 | Light Chain | RASESVDNYGISLMH (SEQ ID NO:18) | RASNLES (SEQ ID NO:13) | QQSNKDPPT (SEQ ID NO:19) |
| | Heavy Chain | SITSGYYWN (SEQ ID NO:15) | MGYISYDGSNN (SEQ ID NO:16) | ARMDWDGDAMDY (SEQ ID NO:20) |
| mHE4 | Light Chain | RASENIYSYLA (SEQ ID NO:21) | NAKTLAE (SEQ ID NO:22) | QNHYGTPYT (SEQ ID NO:23) |
| | Heavy Chain | TFSSYVMS (SEQ ID NO:24) | VATISDGDSSTY (SEQ ID NO:25) | ARDKGHGYDEGAFV Y (SEQ ID NO:26) |
| mHE5 | Light Chain | RASESVDHYGSSFMH (SEQ ID NO:27) | RASNPES (SEQ ID NO:28) | QQSNKDPPT (SEQ ID NO:19) |
| | Heavy Chain | SITSGYYWN (SEQ ID NO:15) | MGYISYDGSNN (SEQ ID NO:16) | ASYDYDGDWFAY (SEQ ID NO:29) |
| mHE6 | Light Chain | SASSSVSYMY (SEQ ID NO:30) | LTSNLAS (SEQ ID NO:31) | QQWSRNPLT (SEQ ID NO:32) |
| | Heavy Chain | TFTDYYMH (SEQ ID NO:33) | IGYINPNNGDAI (SEQ ID NO:34) | ATYDYGGDFYFDV (SEQ ID NO:35) |

### Example 4: Expression of Chimeric Antibodies

The genes encoding the variable region of the heavy chain and light chain obtained in Example 3 were amplified by PCR using PrimeSTAR^{®} Max DNA Polymerase (TAKARA). These nucleic acid sequences of heavy chain variable region and the light chain variable region were then fused with the nucleic acid sequence encoding the human IgG1 heavy chain constant region (amino acid sequence GenBank accession number: AXN93653.1) the nucleic acid sequence encoding the human kappa light chain constant region (amino acid sequence GenBank accession number: AWK57456.1) to generate full-length heavy and light chain genes. The desired genes were separated by agarose gel electrophoresis, purified using a gel extraction kit (Omega), and finally cloned into an expression vector using ClonExpress Ultra One Step Cloning Kit (Vazyme).

After sequencing confirmed the correctness of the expression vectors, the chimeric antibodies were expressed. Corresponding to mHE1-mHE6 heavy and light chains, the following chimeric antibodies cHE1-G1W, cHE2-G1W, cHE4-G1W, cHE5-G1W, cHE6-G1W, and cHE7-G1W were obtained. The performances of the chimeric antibodies were subsequently evaluated.

### Example 5: Analysis of Binding Ability of Chimeric Proteins to Antigens (ELISA)

The binding of chimeric antibodies cHE1-G1W, cHE2-G1W, cHE4-G1W, cHE5-G1W, cHE6-G1W, and cHE7-G1W to human and cynomolgus HER3 extracellular domain proteins was evaluated using ELISA. Human HER3-ECD-His protein and cynomolgus HER3-ECD-His protein (Sino Biological) were coated onto 96-well microplates, incubated with 5% skim milk at room temperature for 1 hour, and washed. Gradient-diluted antibodies were added, incubated at room temperature for 1 hour, and the plates were washed again. HRP-labeled goat anti-human Fc secondary antibody (Jackson) was then added, incubated at room temperature for 1 hour, followed by washing. TMB was used for color development, and absorbance at 450 nm was recorded using a microplate reader.

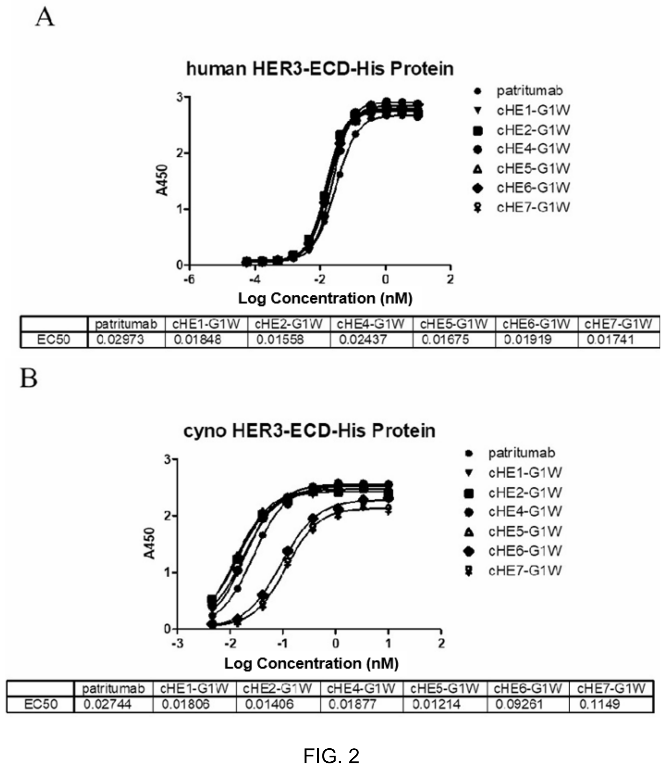

The results, as shown in panel A of FIG. 2, indicated that all chimeric antibodies exhibited binding affinity to human HER3 similar to that of the reference antibody patritumab (WO2007077028). As shown in panel B of FIG. 2, cHE6-G1W and cHE7-G1W bound cynomolgus HER3 with EC50 values 3-4 fold higher than patritumab.

### Example 6: Analysis of Binding Ability of Chimeric Proteins to Antigens (FACS)

FACS was used to evaluate the binding of chimeric antibodies cHE1-G1W, cHE2-G1W, cHE4-G1W, cHE5-G1W, cHE6-G1W, and cHE7-G1W to cells expressing human or cynomolgus HER3. CHO cells overexpressing human HER3 and CHO cells overexpressing cynomolgus HER3 were collected by centrifugation, resuspended in PBS, counted, and added to a 96-well plate. After centrifugation, the supernatant was removed, and gradient-diluted antibodies were added. The cells were incubated at 4°C for 30 minutes, washed twice, and PE-labeled goat anti-human IgG Fc secondary antibody (Jackson) was added. After incubation at 4°C in the dark for 30 minutes, the cells were washed once, resuspended in PBS, and analyzed by flow cytometry.

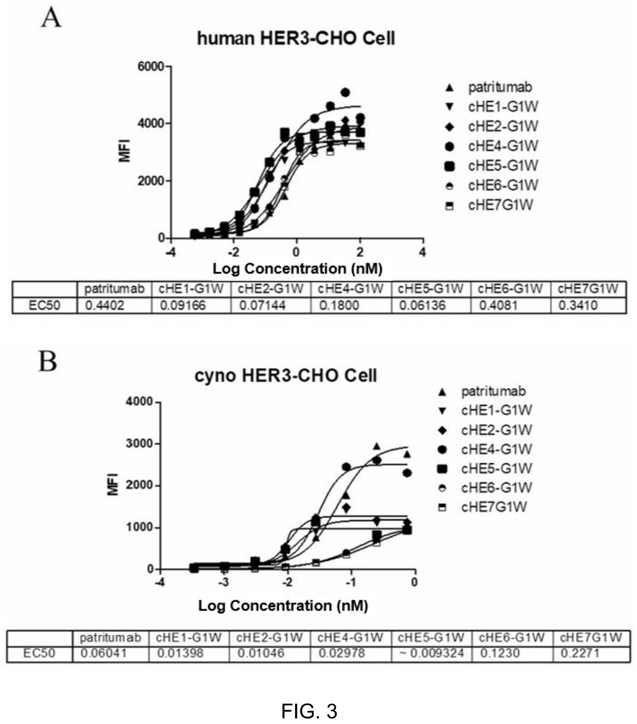

As shown in panel A of FIG. 3, chimeric antibodies cHE1-G1W, cHE2-G1W, cHE4-G1W, and cHE5-G1W bound to human HER3 with EC₅₀ values 2-7 times higher than patritumab, while cHE6-G1W and cHE7-G1W had binding affinity similar to that of patritumab.

As shown in panel B of FIG. 3, cHE4-G1W bound cynomolgus HER3 with an EC₅₀ value 2-fold higher than patritumab, whereas the binding affinity of other chimeric antibodies to cynomolgus HER3 was significantly reduced.

### Example 7: Affinity Analysis of Chimeric Antibodies

The affinity of the six chimeric antibodies to human HER3-ECD-His and cynomolgus HER3-ECD-His proteins was measured using the Octet system. The results are summarized in Table 4.

In terms of the Ka values, all six chimeric antibodies exhibited faster association rate with human and cynomolgus HER3 proteins than patritumab.

In terms of the Kd values, cHE4-G1W exhibited a slower dissociation rate than patritumab with both human and cynomolgus HER3 proteins. cHE6-G1W and cHE7-G1W showed a slower dissociation rate than patritumab with human HER3, but a faster dissociation rate than patritumab with cynomolgus HER3.

In terms of the KD values, cHE4-G1W, cHE6-G1W, and cHE7-G1W had a higher affinity for human HER3-ECD-His compared to patritumab. Additionally, cHE6-G1W and cHE7-G1W showed similar affinity for both human and cynomolgus HER3, demonstrating that the removal of the glycosylation site in cHE6-G1W did not affect its binding affinity for either human HER3 protein or cynomolgus HER3 protein.

**Table 4**

| Antibody ID | Antigen | KD(M) | ka(1/Ms) | Kd(1/s) |
|---|---|---|---|---|
| patritumab | hHER3-ECD-His | 8.69E-10 | 2.58E+05 | 2.24E-04 |
| cHE1-G1W | | 4.88E-09 | 7.28E+05 | 3.56E-03 |
| cHE2-G1W | | 3.57E-09 | 8.61E+05 | 3.08E-03 |
| cHE4-G1W | | 4.09E-10 | 5.73E+05 | 2.34E-04 |
| cHE5-G1W | | 3.56E-09 | 7.10E+05 | 2.53E-03 |
| cHE6-G1W | | 1.14E-12 | 4.50E+05 | 5.11E-07 |
| cHE7-G1W | | 1.31E-12 | 4.25E+05 | 5.57E-07 |
| patritumab | Cyno HER3-ECD-His | 2.34E-09 | 1.31E+05 | 3.06E-04 |
| cHE1-G1W | | 4.01E-09 | 4.23E+05 | 1.70E-03 |
| cHE2-G1W | | 3.25E-09 | 5.07E+05 | 1.65E-03 |
| cHE4-G1W | | 3.10E-10 | 3.74E+05 | 1.16E-04 |
| cHE5-G1W | | 3.58E-09 | 4.29E+05 | 1.54E-03 |
| cHE6-G1W | | 1.25E-09 | 5.65E+05 | 7.06E-04 |
| cHE7-G1W | | 1.36E-09 | 6.72E+05 | 9.12E-04 |

### Example 8: Thermal Stability Analysis of Chimeric Antibodies

The thermal stability of six chimeric antibodies and patritumab was evaluated using Dynamic Light Scattering (DLS). The DLS instrument (Wyatt) measured light scattering of the antibodies as the temperature increased from 25°C to 85°C, and the aggregation temperature (T_{agg}) of each antibody was determined.

The results, as shown in Table 5, indicated that the T_{agg} of all six chimeric antibodies was above 60°C. Specifically, cHE1-G1W, cHE2-G1W, cHE4-G1W, and cHE5-G1W exhibited higher T_{agg} values than patritumab, indicating superior thermal stability compared to patritumab.

**Table 5**

| Antibody ID | T_{agg} (°C) |
|---|---|
| cHE1-G1W | 72.53 |
| cHE2-G1W | 71.72 |
| cHE4-G1W | 68.80 |
| cHE5-G1W | 72.54 |
| cHE6-G1W | 60.82 |
| cHE7-G1W | 61.50 |
| Patritumab | 68.52 |

### Example 9: Epitope Analysis of Chimeric Antibodies

Octet analysis was performed to evaluate the epitopes of cHE1-G1W, cHE2-G1W, cHE4-G1W, cHE5-G1W, cHE7-G1W, and patritumab for binding to human HER3. AMC sensors were first loaded with 10 µg/mL human HER3-ECD protein carrying a mouse Fc tag. The first antibody was then bound until the signal reached saturation, followed by the introduction of a second antibody to assess whether the signal increased. An increase in signal indicated that the first and second antibodies recognized different epitopes on HER3, whereas no change in signal indicated recognition of the same epitope on HER3.

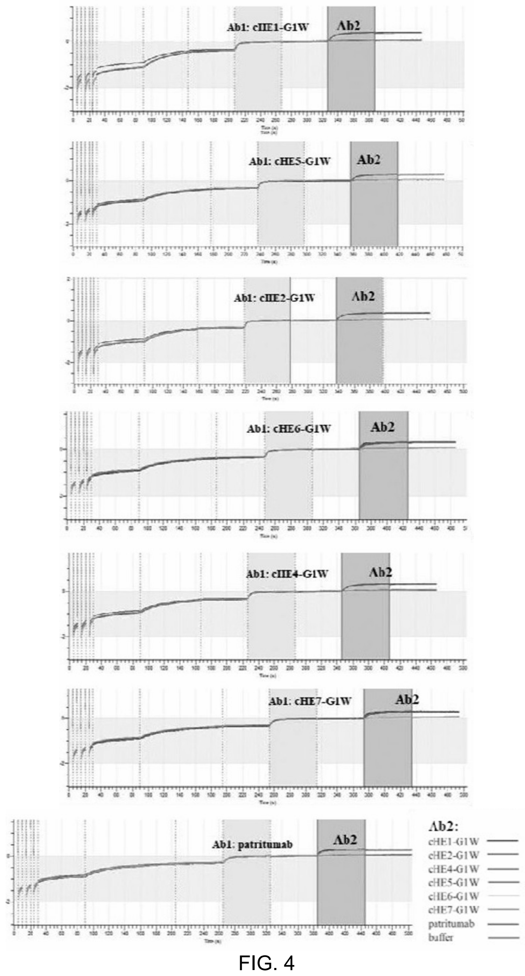

The results, as shown in FIG. 4, indicated that cHE1-G1W, cHE2-G1W, cHE4-G1W, and cHE5-G1W bound to the same epitope, whereas cHE7-G1W bound to a different epitope. None of these antibodies competed with patritumab for binding to human HER3, demonstrating that the chimeric antibodies recognized distinct epitopes from patritumab.

### Example 10: Analysis of Cellular Internalization After Chimeric Antibody Binding to Antigen

The internalization of cHE4-G1W, cHE5-G1W, and cHE7-G1W after binding to human HER3 was assessed. Antibodies at 10 nM were incubated with A375 cells overexpressing human HER3 at 4°C for 1 hour to allow binding, and unbound antibodies were removed after washing. The 0-hour sample was maintained at 4°C, and the samples were transferred to a 37°C incubator. Samples were collected at 1, 2, and 3 hours, respectively, and PE-labeled goat anti-human Fc secondary antibody (Jackson) was added. After incubation at 4°C for 30 minutes, the unbound secondary antibody was removed by washing. Internalization was analyzed by flow cytometry.

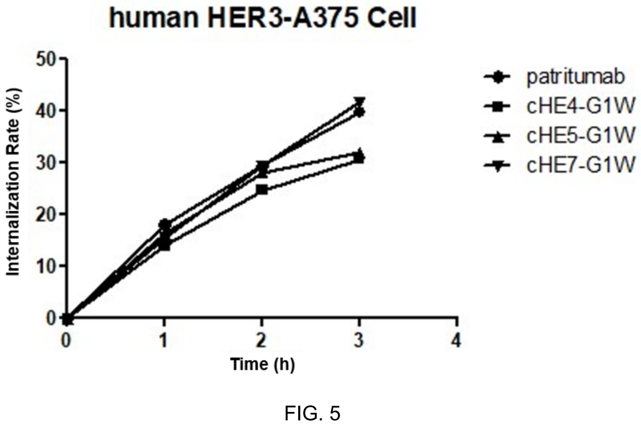

As shown in of FIG. 5, cHE4-G1W, cHE5-G1W, and cHE7-G1W were internalized after binding to HER3 on the surface of A375 cells, with cHE7-G1W exhibiting an internalization rate similar to patritumab.

### Example 11: Preparation of Conjugated Drugs Based on Chimeric Antibodies and Tumor Cell Killing Analysis

Chimeric antibodies cHE4-G1W, cHE5-G1W, and cHE7-G1W were used for preparing antibody-drug conjugates (ADCs), and the cytotoxicity of the antibody-drug conjugates against A375 cells overexpressing human HER3 and HER3-expressing OVCAR3 tumor cells was evaluated. Chimeric antibodies were first treated with TCEP (Sigma) to reduce interchain disulfide bonds, and then maleimide-GGFG-DXd (MedChemExpress) was added for conjugation. The resulting ADCs were purified using a desalting column to remove excess small-molecule payload. The obtained antibody-drug conjugates were designated as cHE4-G1W-DXd, cHE5-G1W-DXd, cHE7-G1W-DXd, and patritumab-DXd, with IgG-DXd serving as a negative control. Hydrophobic interaction chromatography revealed antibody-to-DXd ratios of 1:7 for cHE4, 1:6.9 for cHE5, 1:7.7 for cHE7, 1:8 for patritumab, and 1:7.5 for IgG.

A375 and OVCAR3 cells were seeded in 96-well plates at 5,000 cells per well, and gradient-diluted ADCs (cHE4-G1W-DXd, cHE5-G1W-DXd, cHE7-G1W-DXd, patritumab-DXd, and IgG-DXd) were added. After incubation at 37°C for 5 days, cell viability was measured using CellTiter-Glo (Promega) to evaluate cytotoxicity of the ADCs.

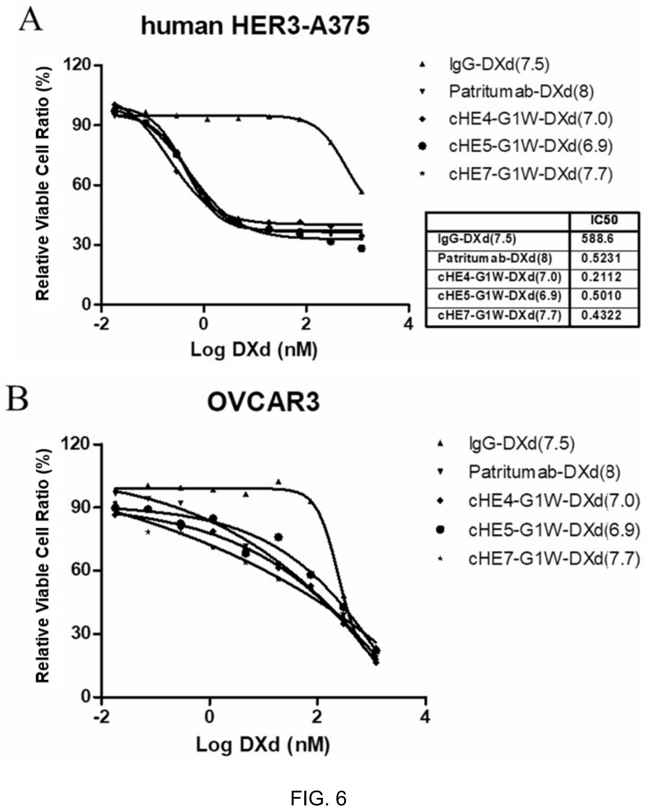

The results, as shown in panels A and B of FIG. 6, indicated that cHE4-G1W-DXd, cHE5-G1W-DXd, and cHE7-G1W-DXd exhibited significant killing effects on A375 cells, with cHE4-G1W-DXd showing approximately 2-fold higher cytotoxicity than patritumab-DXd. For OVCAR3 cells, cHE4-G1W-DXd, cHE5-G1W-DXd, and cHE7-G1W-DXd demonstrated better cytotoxicity compared to the negative control IgG-DXd, with comparable cytotoxicity to patritumab-DXd.

### Example 12: Preparation of Humanized Antibodies

Based on the experimental results from Examples 5 to 11, the chimeric antibody cHE4-G1W was selected for humanization. The variable regions of the heavy and light chains of cHE4-G1W were analyzed using the IMGT database, and the CDR1, CDR2, and CDR3 regions critical for antigen binding were identified as shown in Table 3. During humanization, the CDR regions were retained, and the murine FR regions were replaced with the most homologous FR regions derived from human germline antibodies, ultimately generating a humanized antibody. The variable regions of the heavy and light chains of the humanized antibody are shown in Table 6.

**Table 6**

| Humanized Antibody ID | Monoclonal Antibody Variable Region |
|---|---|
| hHE4 Light Chain (SEQ ID NO:36) | DIQMTQSPSSLSASVGDRVTITCRASENIYSYLAWYQQKPGKA PKLLIYNAKTLAEGVPSRFSGSGSGTDFTLTISSLQPEDFATYYC QNHYGTPYTFGQGTKLEIKR |
| | CDR1: RASENIYSYLA (SEQ ID NO:21) |
| | CDR2: NAKTLAE (SEQ ID NO:22) |
| | CDR3: QNHYGTPYT (SEQ ID NO:23) |
| hHE4 Heavy Chain (SEQ ID NO:37) | QVQLVESGGGLVKPGGSLRLSCAASGFTFSSYVMSWVRQAPG KGLEWVATISDGDSSTYYADSVKGRFTISRDNAKNSLYLQMNSL RAEDTAVYYCARDKGHGYDEGAFVYWGQGTLVTVSS |
| | CDR1: TFSSYVMS (SEQ ID NO:24) |
| | CDR2: VATISDGDSSTY (SEQ ID NO:25) |
| | CDR3: ARDKGHGYDEGAFVY (SEQ ID NO:26) |

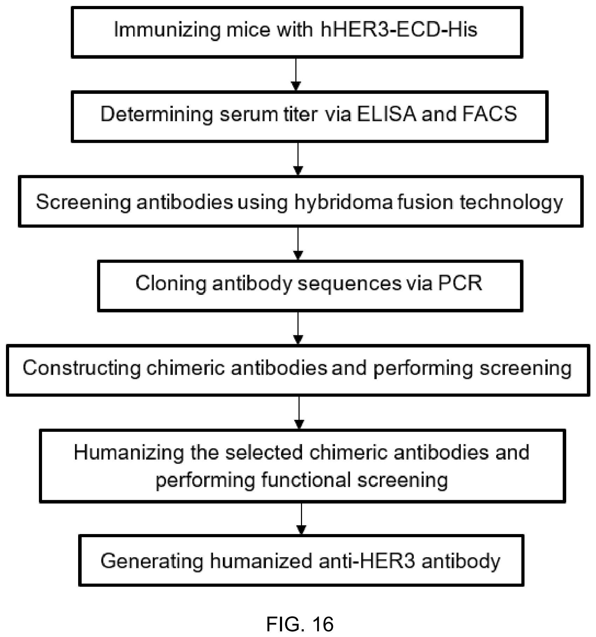

The nucleotide sequences corresponding to amino acid sequences of the variable regions of the humanized hHE4 antibody were obtained, and codonoptimization was performed for gene synthesis. The nucleotide sequences of the variable regions were then fused to the nucleotide sequences of the human IgG1 heavy and light chain constant regions and inserted into expression vectors. After sequence verification, plasmids were transfected into ExpiCHO cells for expression. The humanized antibody hHE4-G1W was purified using Protein A. The schematic process for obtaining the humanized antibody is shown in FIG. 16.

### Example 13: Affinity Analysis of Humanized Antibody

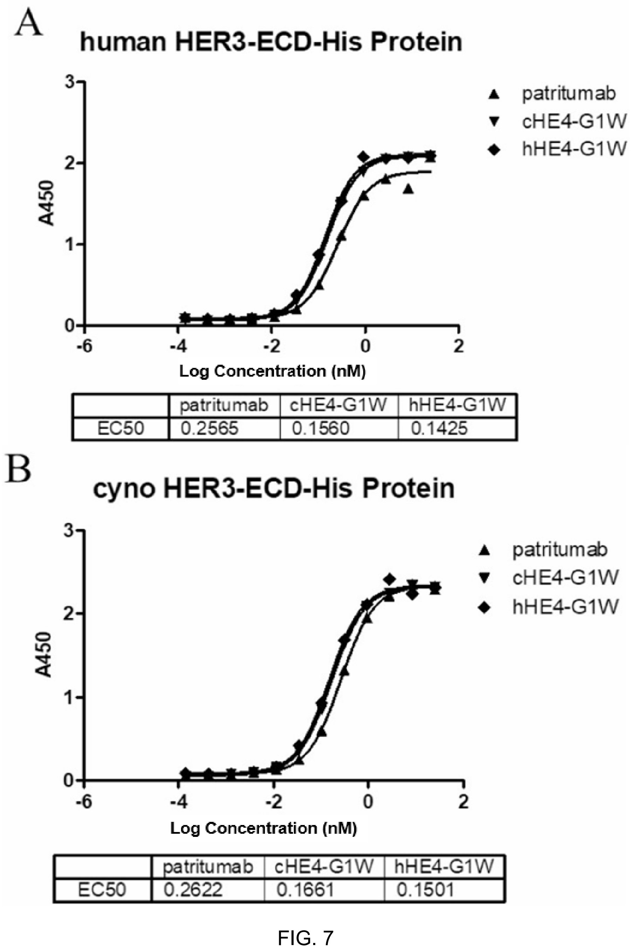

The binding affinities of the humanized antibody hHE4-G1W to human and cynomolgus HER3 were evaluated by ELISA, and the results were compared with that of the chimeric antibody cHE4-G1W. Human HER3-ECD-His and cyno HER3-ECD-His proteins were coated onto microplates, blocked with 5% skim milk, incubated at room temperature for 1 hour, and washed. Gradient-diluted antibodies were added and incubated for 1 hour at room temperature, followed by washing. HRP-labeled goat anti-human Fc secondary antibody (Jackson) was added, incubated at room temperature for 1 hour, and washed. TMB was used for color development, and absorbance at 450 nm was measured. As shown in panels A and B of FIG. 7, after humanization, hHE4-G1W maintained better binding affinity for both human and cynomolgus HER3 when compared to patritumab, and exhibited a binding profile similar to that of the chimeric antibody cHE4-G1W.

### Example 14: Analysis of Antigen-Binding Ability of Humanized Antibody

Flow cytometry (FACS) was used to compare the cell-binding affinity of the humanized antibody hHE4-G1W with that of the chimeric antibody cHE4-G1W toward cells expressing human HER3 or cynomolgus monkey HER3. The procedure was the same as in Example 6.

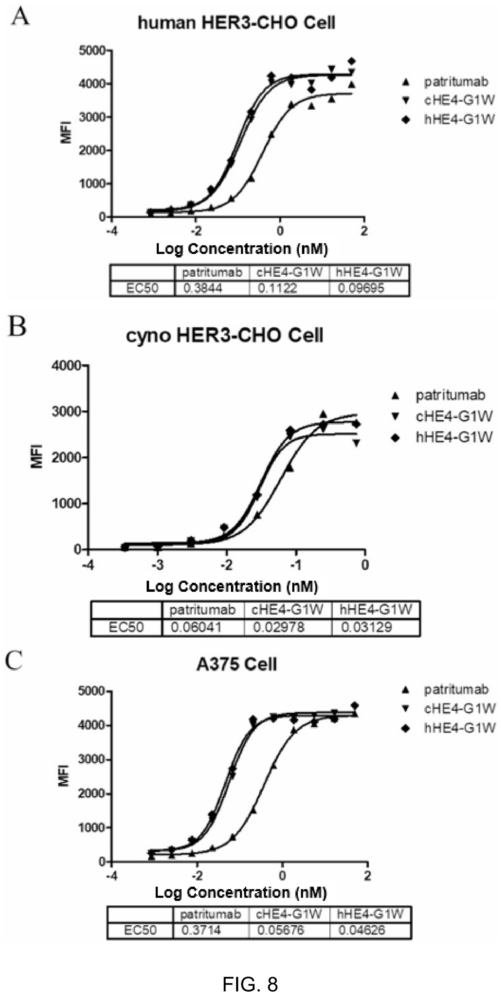

As shown in FIG. 8, whether using CHO cells overexpressing human HER3 (panel A), parental A375 cells (panel C), or CHO cells overexpressing cynomolgus monkey HER3 (panel B), humanization did not alter the cell-surface binding of hHE4-G1W relative to cHE4-G1W to either human or cynomolgus monkey HER3. Furthermore, hHE4-G1W bound to human HER3 with an EC₅₀ value 4-8 times lower than patritumab.

### Example 15: Affinity Analysis of Humanized Antibody

The affinity of humanized antibody hHE4-G1W to human HER3-ECD-His and cyno HER3-ECD-His was analyzed using the Octet system. The results are shown in Table 7. In terms of the Ka, Kd, and KD values, it was indicated that the affinity of humanized antibody hHE4-G1W to human and cyno HER3 remained similar to that of chimeric antibody cHE4-G1W, while the KD value of humanized antibody hHE4-G1W was approximately 10-fold lower than that of patritumab.

**Table 7**

| Antibody ID | Antigen | KD(M) | ka(1/Ms) | Kd(1/s) |
|---|---|---|---|---|
| cHE4-G1W | hHER3-ECD-His | 2.33E-10 | 4.82E+05 | 1.12E-04 |
| hHE4-G1W | | 1.99E-10 | 4.95E+05 | 9.84E-05 |
| patritumab | | 1.33E-09 | 2.22E+05 | 2.96E-04 |
| cHE4-G1W | Cyno HER3-ECD-His | 2.27E-10 | 4.25E+05 | 9.63E-05 |
| hHE4-G1W | | 2.08E-10 | 4.39E+05 | 9.11E-05 |
| patritumab | | 1.92E-09 | 1.94E+05 | 3.71E-04 |

### Example 16: Epitope Analysis of Humanized Antibody

An ELISA was performed to determine which HER3 extracellular domain(s) are recognized by the humanized antibody hHE4-G1W. According to literature (Acta Crystallogr F Struct Biol Commun. 2021 Jul 1;77(Pt 7):192-201), the extracellular domain of human HER3 includes four subdomains: subdomain I (corresponding to amino acid residues 56-183), subdomain II (corresponding to amino acid residues 184-308), subdomain III (corresponding to amino acid residues 309-500), and subdomain IV (corresponding to amino acid residues 501-643). Fusion proteins including subdomains I-III (56-500) or I-IV (56-643), each carrying an mFc tag, were expressed and purified for ELISA. Goat anti-human Fc antibody (Jackson) was coated on microplates for 3 hours. After washing, the plate was blocked with 5% skim milk for 1 hour. After washing, hHE4-G1W (2 µg/mL) and patritumab (2 µg/mL) were added and incubated at room temperature for 1 hour, followed by another washing. Gradient-diluted mFc-tagged fusion protein, including HER3 I-III or I-IV was added and incubated for 1 hour. HRP-labeled rabbit anti-mouse Fc secondary antibody (Thermo Fisher) was then added, incubated for 1 hour, washed, and developed with TMB. Absorbance was measured at 450 nm.

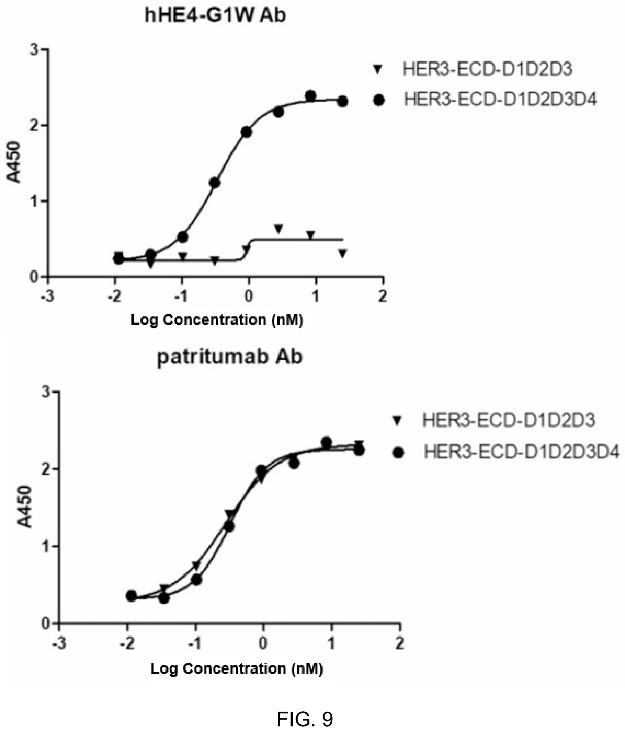

As shown in FIG. 9, hHE4-G1W bound to the I-IV subdomains but not the I-III subdomains of HER3, indicating that the binding epitope of hHE4-G1W is located within subdomain IV. In contrast, patritumab bound to both I-III and I-IV subdomains, indicating the binding epitope of patritumab is located within subdomains I-III.

Therefore, hHE4-G1W and patritumab targeted distinct epitopes on the human HER3 protein.

### Example 17: Binding Specificity Analysis of Humanized Antibody

To confirm that hHE4-G1W binds specifically to human HER3, an ELISA was performed to test whether hHE4-G1W exhibits binding to other human EGFR family members, namely EGFR, HER2, and HER4. Human EGFR-ECD-His, Human HER2-ECD-His, and Human HER4-ECD-His proteins were coated onto microplates, blocked with 5% skim milk at room temperature for 1 hour, and washed. 25 nM antibodies, serially diluted 3-fold down to 0.011 nM, were added and incubated at room temperature for 1 hour, followed by washing. HRP-labeled goat anti-human Fc secondary antibody (Jackson) was added, incubated at room temperature for 1 hour, and washed. TMB was used for color development, and absorbance at 450 nm was measured.

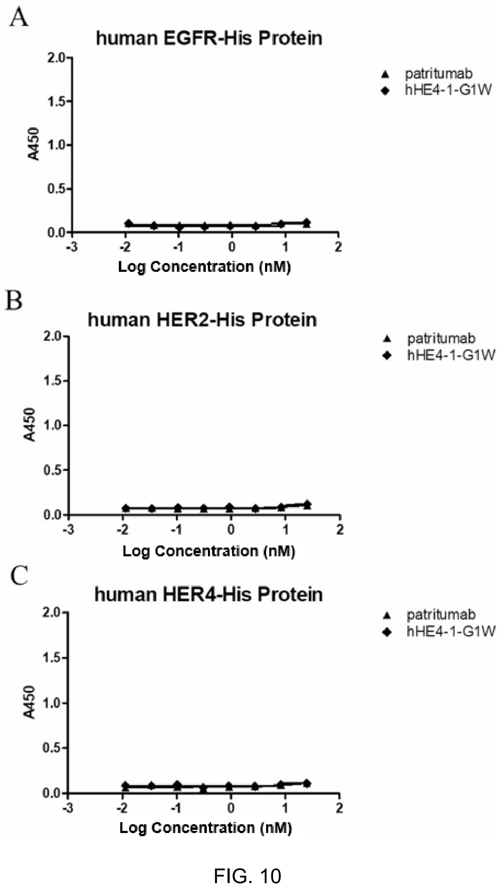

As shown in panels A-C of FIG. 10, hHE4-G1W and patritumab did not bind to EGFR, HER2, or HER4, indicating that hHE4-G1W binds specifically to human HER3.

### Example 18: Thermal Stability Analysis of Humanized Antibody

The thermal stability of humanized antibody hHE4-G1W was evaluated using dynamic light scattering (DLS) following the method described in Example 8. The results are shown in Table 8. Compared with the chimeric antibody cHE4-G1W, the Tgg of hHE4-G1W increased from 69.29°C to 75.54°C. Additionally, humanized antibody hHE4-G1W showed significantly improved thermal stability compared with patritumab.

**Table 8**

| Antibody ID | T_{agg} (°C) |
|---|---|
| cHE4-G1W | 69.29 |
| hHE4-G1W | 75.54 |
| patritumab | 69.12 |

### Example 19: Analysis of Cellular Internalization After Humanized Antibody Binding to Antigen

A cell imaging microplate detection system was used to monitor the internalization of hHE4-G1W, hHE4-G1W-DXd, patritumab, and patritumab-DXd after binding to HER3 on the surface of A375 tumor cells. hHE4-G1W, hHE4-G1W-DXd, patritumab, and patritumab-DXd (each 100 nM) were incubated with HER3-overexpressing A375 cells at 4°C for 30 minutes, respectively. Unbound antibodies were removed by washing, and PE-labeled goat anti-human Fc secondary antibody (Jackson) was added. The mixture was incubated at 4°C for 30 minutes, and then the unbound secondary antibody was removed by washing. When the antibodies were bound to the surface of A375 cells and had not yet undergone internalization, these samples were designated as the 0h samples. Fluorescence images were acquired using a Biotek Cytation 1 imaging reader. Cells were then incubated at 37°C for 3 hours and imaged again.

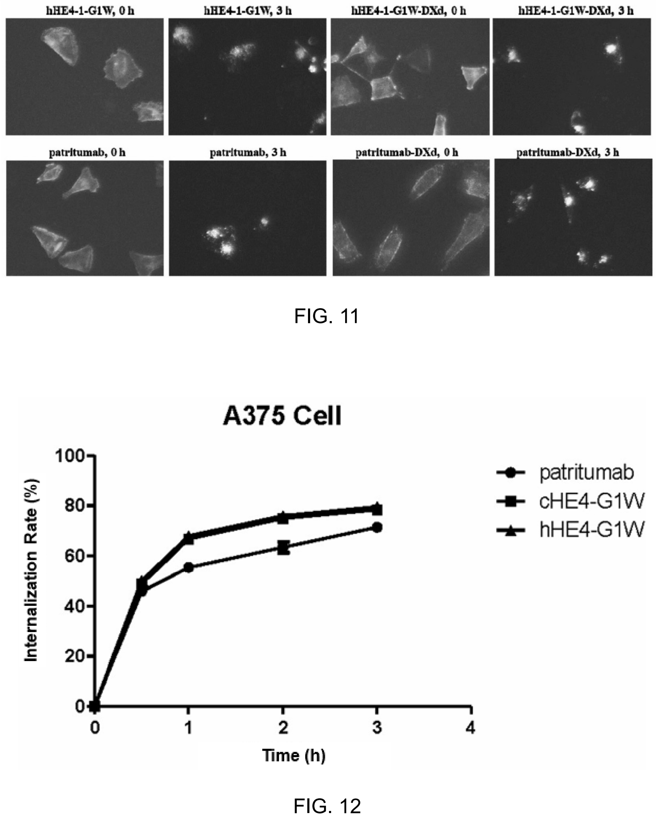

As shown in FIG. 11, at 0 hour, fluorescence was observed on the surface of A375 cells, indicating that hHE4-G1W, hHE4-G1W-DXd, patritumab, and patritumab-DXd all bound to HER3 expressed on the cell surface. After 3 hours, surface fluorescence decreased, while intracellular fluorescence increased, indicating that hHE4-G1W, hHE4-G1W-DXd, patritumab, and patritumab-DXd were internalized into the A375 cells.

### Example 20: Analysis of Cellular Internalization After Humanized Antibody Binding to Antigen

FACS was used to evaluate the internalization rate of humanized antibody hHE4-G1W, chimeric antibody cHE4-G1W4, and reference antibody patritumab after binding to HER3 expressed on the surface of A375 cells. The method was the same as that in Example 10.

As shown in FIG. 12, after binding to HER3 expressed on the surface of A375 cells, hHE4-G1W and cHE4-G1W4 displayed similar internalization rates during 3-hour incubation at 37°C, which were faster than the internalization rate of patritumab.

### Example 21: Preparation of Conjugated Drugs Based on Humanized Antibodies and Tumor Killing Analysis

The cytotoxicity of antibody-drug conjugates based on hHE4-G1W against HER3-expressing HCC1569, A375, and JIMT-1 tumor cells was tested. Antibody-drug conjugates were prepared as described in Example 11. Hydrophobic interaction chromatography indicated the antibody-to-Dxd ratios for cHE4-G1W-DXd, hHE4-G1W-DXd, patritumab-DXd, and negative control IgG-DXd were 1:7, 1:8, 1:7.8, and 1:8, respectively.

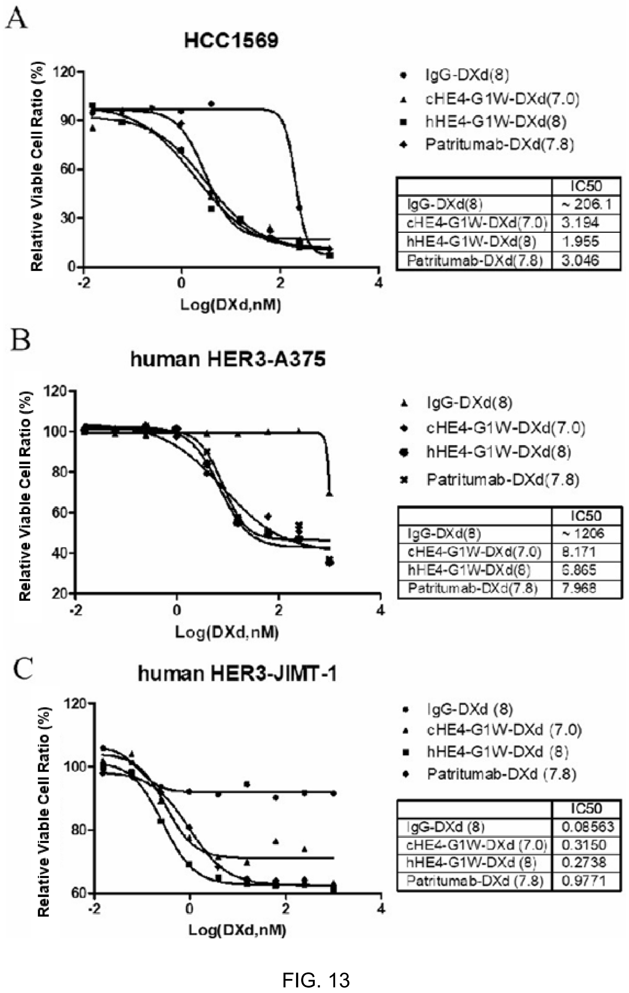

As shown in panels A-C of FIG. 13, hHE4-G1W-DXd, cHE4-G1W-DXd, and patritumab-DXd all exhibited significant cytotoxicity against HER3-expressing HCC1569 cells, as well as against human HER3-overexpressing A375 and JIMT-1 cells. Notably, hHE4-G1W-DXd demonstrated superior cytotoxicity compared with patritumab-DXd against human HER3-overexpressing JIMT-1 cells.

### Example 22: Tumor Inhibition Analysis of Humanized Antibody-Drug Conjugates in SW620 Tumor-Bearing Mice

The anti-tumor efficacy of hHE4-G1W-DXd and cHE5-G1W-DXd was evaluated in SW620 tumor-bearing mice. Female SCID mice were subcutaneously injected with SW620 cells in the dorsal region to establish the xenograft model. After tumor establishment, mice were randomly divided into nine groups and treated with a single dose. Three groups served as negative controls: one group received PBS, and two groups received IgG-DXd at low dose (3 mg/kg) and high dose (6 mg/kg), respectively. Experimental groups were administered with hHE4-G1W-DXd, cHE5-G1W-DXd, or patritumab-DXd at 3 mg/kg or 6 mg/kg. The average Dxd-to-antibody ratio was 8. Mouse body weights and tumor volumes were measured.

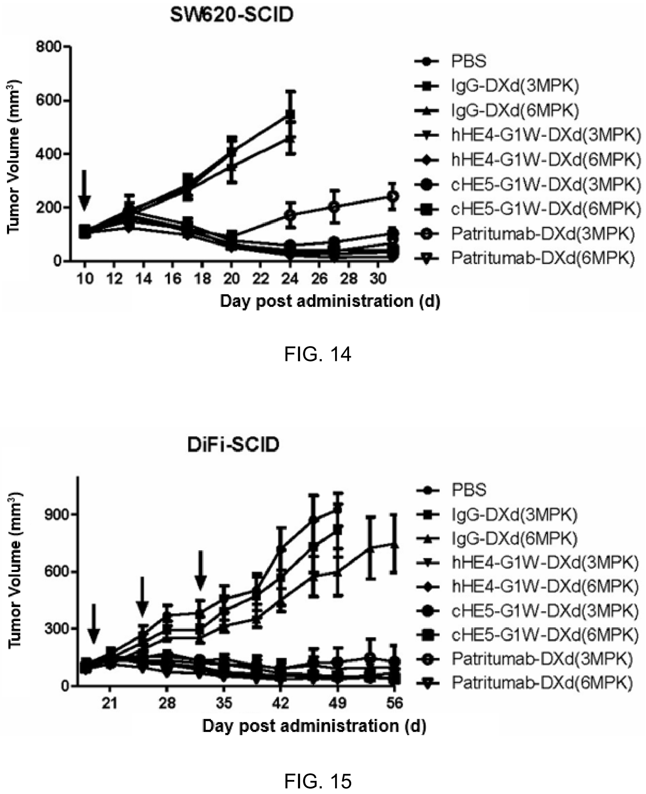

As shown in FIG. 14, compared with PBS and IgG-DXd control groups, all experimental groups exerted an inhibitory effect on the SW620 tumor. Notably, 3 mg/kg patritumab-DXd showed significant tumor growth at day 14 postadministration. In contrast, 3 mg/kg hHE4-G1W-DXd prevented tumor growth, and administration of 6 mg/kg hHE4-G1W-DXd resulted in complete tumor regression in one mouse. These results indicated that hHE4-G1W-DXd had superior tumor inhibition against SW620 tumor compared with patritumab-DXd.

### Example 23: Tumor Inhibition Analysis of Humanized Antibody-Drug Conjugates in DiFi Tumor-Bearing Mice

The anti-tumor efficacy of hHE4-G1W-DXd and cHE5-G1W-DXd was evaluated in DiFi tumor-bearing mice. Female SCID mice were subcutaneously injected with DiFi cells in the dorsal region to establish the xenograft model. After tumor establishment, mice were randomly divided into nine groups and treated once per week for three weeks. Three groups served as negative controls: one group received PBS, and two groups received IgG-DXd at low dose (3 mg/kg) and high dose (6 mg/kg), respectively. Experimental groups were administered with hHE4-G1W-DXd, cHE5-G1W-DXd, or patritumab-DXd at 3 mg/kg or 6 mg/kg. The average Dxd-to-antibody ratio was 8. Mouse body weights and tumor volumes were measured.

As shown in FIG. 15, compared with PBS and IgG-DXd control groups, all experimental groups exerted an inhibitory effect on the DiFi tumor. At low dose of 3 mg/kg, hHE4-G1W-DXd and cHE5-G1W-DXd showed superior tumor inhibition compared with patritumab-DXd. At high dose of 6 mg/kg, the tumor inhibition effects were comparable among the experimental groups. These results indicated that hHE4-G1W-DXd and cHE5-G1W-DXd exhibited equivalent or improved tumor inhibition against DiFi tumors compared with patritumab-DXd.

The above examples illustrate several embodiments of the present disclosure in detail. However, they should not be interpreted as limiting the scope of the present disclosure. It should be noted that, for those skilled in the art, various modifications and improvements can be made without departing from the inventive concept of the present disclosure, and these also shall fall within the scope of the present disclosure. Therefore, the scope of the present disclosure should be defined by the appended claims. All references cited in this application are incorporated by reference as if each reference were individually and expressly incorporated by reference in its entirety.

## Claims

1. An anti-HER3 antibody, comprising a light chain variable region and a heavy chain variable region, wherein the anti-HER3 antibody is one of:
antibody (a), whose amino acid sequences of CDR1, CDR2, and CDR3 of the light chain variable region are as shown in SEQ ID NOs:21, 22, and 23, respectively; and amino acid sequences of CDR1, CDR2, and CDR3 of the heavy chain variable region are as shown in SEQ ID NOs:24, 25, and 26, respectively;
antibody (c), whose amino acid sequences of CDR1, CDR2, and CDR3 of the light chain variable region are as shown in SEQ ID NOs:30, 31, and 32, respectively; and amino acid sequences of CDR1, CDR2, and CDR3 of the heavy chain variable region are as shown in SEQ ID NOs:33, 34, and 35, respectively;
antibody (d), whose amino acid sequences of CDR1, CDR2, and CDR3 of the light chain variable region are as shown in SEQ ID NOs:12, 13, and 14, respectively; and amino acid sequences of CDR1, CDR2, and CDR3 of the heavy chain variable region are as shown in SEQ ID NOs:15, 16, and 17, respectively;
antibody (e), whose amino acid sequences of CDR1, CDR2, and CDR3 of the light chain variable region are as shown in SEQ ID NOs:18, 13, and 19, respectively; and amino acid sequences of CDR1, CDR2, and CDR3 of the heavy chain variable region are as shown in SEQ ID NOs:15, 16, and 20, respectively; and
antibody (f), whose amino acid sequences of CDR1, CDR2, and CDR3 of the light chain variable region are as shown in SEQ ID NOs:27, 28, and 19, respectively; and amino acid sequences of CDR1, CDR2, and CDR3 of the heavy chain variable region are as shown in SEQ ID NOs:15, 16, and 29, respectively.

2. The antibody of claim 1, wherein the anti-HER3 antibody is a monoclonal antibody, a single-chain variable fragment antibody, a domain antibody, an Fab fragment, an Fd fragment, an Fv fragment, or an F(ab')₂ fragment; or the anti-HER3 antibody is a humanized antibody, a chimeric antibody, or a murine antibody.

3. The antibody of claim 1, wherein the anti-HER3 antibody is:
(1) an antibody comprising (i) a light chain variable region having an amino acid sequence as shown in SEQ ID NO:36 or a light-chain variable region having at least 85% sequence identity to the amino acid sequence as shown in SEQ ID NO: 36, and (ii) a heavy chain variable region having an amino acid sequence as shown in SEQ ID NO:37 or a heavy chain variable region having at least 85% sequence identity to the amino acid sequence as shown in SEQ ID NO: 37;
(2) an antibody comprising (i) a light chain variable region having an amino acid sequence as shown in SEQ ID NO:5 or a light-chain variable region having at least 85% sequence identity to the amino acid sequence as shown in SEQ ID NO: 5, and (ii) a heavy chain variable region having an amino acid sequence as shown in SEQ ID NO:6 or a heavy chain variable region having at least 85% sequence identity to the amino acid sequence as shown in SEQ ID NO: 6;
(3) an antibody comprising (i) a light chain variable region having an amino acid sequence as shown in SEQ ID NO:9 or a light-chain variable region having at least 85% sequence identity to the amino acid sequence as shown in SEQ ID NO: 9, and (ii) a heavy chain variable region having an amino acid sequence as shown in SEQ ID NO:10 or a heavy chain variable region having at least 85% sequence identity to the amino acid sequence as shown in SEQ ID NO: 10;
(4) an antibody comprising (i) a light chain variable region having an amino acid sequence as shown in SEQ ID NO:9 or a light-chain variable region having at least 85% sequence identity to the amino acid sequence as shown in SEQ ID NO: 9, and (ii) a heavy chain variable region having an amino acid sequence as shown in SEQ ID NO:11 or a heavy chain variable region having at least 85% sequence identity to the amino acid sequence as shown in SEQ ID NO: 11;
(5) an antibody comprising (i) a light chain variable region having an amino acid sequence as shown in SEQ ID NO:1 or a light-chain variable region having at least 85% sequence identity to the amino acid sequence as shown in SEQ ID NO: 1, and (ii) a heavy chain variable region having an amino acid sequence as shown in SEQ ID NO:2 or a heavy chain variable region having at least 85% sequence identity to the amino acid sequence as shown in SEQ ID NO: 2;
(6) an antibody comprising (i) a light chain variable region having an amino acid sequence as shown in SEQ ID NO:3 or a light-chain variable region having at least 85% sequence identity to the amino acid sequence as shown in SEQ ID NO: 3, and (ii) a heavy chain variable region having an amino acid sequence as shown in SEQ ID NO:4 or a heavy chain variable region having at least 85% sequence identity to the amino acid sequence as shown in SEQ ID NO: 4; or
(7) an antibody comprising (i) a light chain variable region having an amino acid sequence as shown in SEQ ID NO:7 or a light-chain variable region having at least 85% sequence identity to the amino acid sequence as shown in SEQ ID NO: 7, and (ii) a heavy chain variable region having an amino acid sequence as shown in SEQ ID NO:8 or a heavy chain variable region having at least 85% sequence identity to the amino acid sequence as shown in SEQ ID NO: 8.

4. A polynucleotide, wherein the polynucleotide encodes the antibody according to any one of claims 1-3.

5. A construct, comprising the polynucleotide of claim 4.

6. An antibody expression system, comprising the construct of claim 5 or a genome into which the polynucleotide of claim 4 is exogenously integrated.

7. A method for preparing the anti-HER3 antibody according to any one of claims 1-3, comprising performing expression using the antibody expression system of claim 6 under conditions suitable for expression, to obtain the anti-HER3 antibody.

8. A use of the antibody according to any one of claims 1-3, for:
(i) preparing an anti-tumor drug that specifically targets tumor cells expressing HER3;
(ii) preparing an antibody-drug conjugate or an immunoconjugate;
(iii) preparing a bispecific or multispecific antibody;
(iv) preparing a reagent for diagnosing a tumor that expresses HER3; or
(v) preparing immune cells modified with a chimeric antigen receptor.

9. An immunoconjugate, comprising the antibody according to any one of claims 1-3 and a functional molecule connected thereto.

10. The immunoconjugate of claim 9, wherein the functional molecule comprises a tumor inhibitor; a molecule targeting tumor-associated surface markers; a cytotoxin; a radioactive isotope; a biologically active protein; a molecule or a detectable label, each targeting immune cell surface markers; an extracellular hinge region, a transmembrane region and an intracellular signaling region based on chimeric antigen receptor technology; or a combination thereof.

11. The immunoconjugate of claim 10, wherein:
the tumor inhibitor is an anti-tumor toxin or an anti-tumor cytokine; or
the molecule targeting tumor-associated surface markers is an antibody or a ligand that binds to tumor-associated surface markers; or
the molecule targeting immune cell surface markers is an antibody or a ligand that binds to immune cell surface markers.

12. The immunoconjugate of claim 11, wherein:
the anti-tumor toxin comprises a toxin acting on microtubules; a toxin acting on DNA or a derivative thereof; a compound or a derivative thereof that acts on intracellular metabolism, transcription, translation, or signal transduction; or
the anti-tumor cytokine comprises IL-2, IL-12, IL-15, IFN-beta, TNF-alpha, or a variant thereof; or
the antibody that binds to tumor-associated surface markers is an antibody recognizing an antigen other than HER3, wherein the antigen other than HER3 comprises EGFR, EGFRvIII, mesothelin, HER2, EphA2, cMet, EpCAM, MUC1, MUC16, CEA, Claudin 18.2, Claudin 6, WT1, NY-ESO-1, MAGE 3, CD47, ASGPR1, or CDH16; or
the antigen for the antibody that binds to immune cell surface markers comprises CD3, CD4, CD8, PD-1, PD-L1, LAG-3, TIM-3, 4-1BB, OX40, ICOS, B7-H3, CD16, CTLA-4, TIGIT, VISTA, GITR, CD27, SIRPα, CD32, CD64, ILT2, ILT4, NKG2A, NKG2D, or KIR.

13. The immunoconjugate of claim 12, wherein
the toxin acting on microtubules comprises monomethyl auristatin or maytansinoid; or
the toxin acting on DNA comprises DXd, Duocarmycin, Calicheamicin, Pyrrolobenzodiazepines, or SN-38.

14. The immunoconjugate of claim 11 or 12, wherein the anti-tumor toxin is connected to the antibody according to any one of claims 1-3 via a linker, wherein the linker comprises a maleimide-GGFG peptide linker, a mc-Val-Cit-PAB, or a mc-Val-Ala-PAB.

15. A pharmaceutical composition, comprising the antibody according to any one of claims 1-3 or the immunoconjugate according to any one of claims 9-14.

16. A use of the antibody according to any one of claims 1-3, the immunoconjugate according to any one of claims 9-14, or the pharmaceutical composition according to claim 15, in the preparation of a formulation, a kit, or a package for diagnosing or treating a tumor; wherein the tumor is a HER3-related tumor.

17. The use of claim 16, wherein the tumor comprises breast cancer, colon cancer, lung cancer, pancreatic cancer, skin cancer, gastric cancer, or ovarian cancer.

18. The use of claim 17, wherein the skin cancer is melanoma.

19. A kit or package, comprising the antibody according to any one of claims 1-3, the immunoconjugate according to any one of claims 9-14, or the pharmaceutical composition according to claim 15.
